(19) Europäisches Patentamt
European Patent Office
Office européen des brevets

(11) **EP 4 751 718 A1**

(12) **EUROPEAN PATENT APPLICATION**
published in accordance with Art. 153(4) EPC

(43) Date of publication:
**03.06.2026 Bulletin 2026/23**

(21) Application number: **24845302.9**

(22) Date of filing: **26.06.2024**

(51) International Patent Classification (IPC):
***A61K 9/16*** *(2006.01)* ***A61K 31/713*** *(2006.01)*
***A61K 31/7088*** *(2006.01)* ***A61K 47/10*** *(2017.01)*
***A61K 47/24*** *(2006.01)* ***A61K 47/28*** *(2006.01)*
***A61K 47/32*** *(2006.01)*

(52) Cooperative Patent Classification (CPC):
**A61K 9/16; A61K 31/7088; A61K 31/713; A61K 47/10; A61K 47/24; A61K 47/28; A61K 47/32**

(86) International application number:
**PCT/JP2024/023226**

(87) International publication number:
**WO 2025/022920 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.07.2023 JP 2023121431**

(71) Applicant: **Nippon Shokubai Co., Ltd.**
**Osaka-shi, Osaka 541-0043 (JP)**

(72) Inventor: **IMASE, Masato**
**Suita-shi, Osaka 564-0034 (JP)**

(74) Representative: **Winter, Brandl - Partnerschaft mbB**
**Alois-Steinecker-Straße 22**
**85354 Freising (DE)**

Remarks:
The complete document including Reference Table(s) and the Sequence Listing(s) can be downloaded from the EPO website

(54) **LIPID NANOPARTICLES**

(57) Provided is a lipid nanoparticle that achieves sufficient lipid nanoparticle intake into a cell and is capable of efficiently achieving expression of a payload in the lipid nanoparticle. A lipid nanoparticle containing an amphiphilic compound, wherein the amphiphilic compound includes: (i) a site (I) containing a constituent unit (A) derived from a monomer (a) having two or more hydroxy groups in a molecule and having from 2 to 10 carbon atoms constituting a side chain among carbon atoms of the constituent unit; and (ii) a hydrocarbon group having 8 or more carbon atoms.

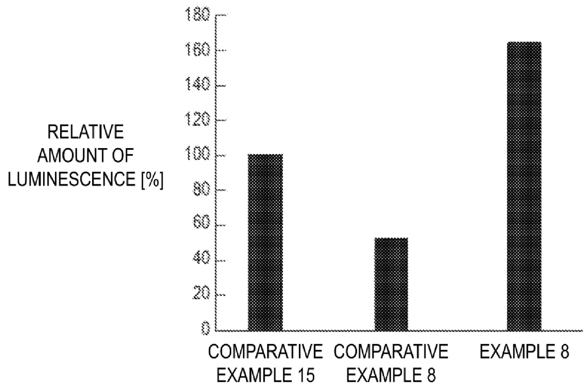


FIG. 1

EP 4 751 718 A1

## Description

Technical Field

The present invention relates to lipid nanoparticles.

Background Art

In recent years, formulations based on drug delivery systems (DDS) (DDS formulations) have been actively developed.

Lipid nanoparticles (LNP) are attracting attention as pharmaceutical carriers for the nucleic acid pharmaceutical DDS because they are capable of delivering nucleic acids into cells. To retain lipid nanoparticles in blood for a certain period of time, lipids modified with polyethylene glycol (PEG) have been used in lipid nanoparticles encapsulating nucleic acids (for example, Patent Document 1).

Citation List

Patent Literature

Patent Document 1: JP 2020-532528 T

Summary of Invention

Technical Problem

An object of the present invention is to provide a lipid nanoparticle that achieves sufficient lipid nanoparticle intake into a cell and is capable of improving the intake efficiency of a payload in the lipid nanoparticle into the cell.

Solution to Problem

Preferred configurations of the lipid nanoparticle of the present invention are described in the following (1) to (17) and the like.

(1) A lipid nanoparticle containing an amphiphilic compound,
wherein the amphiphilic compound includes:

(i) a site (I) containing a constituent unit (A) derived from a monomer (a) having two or more hydroxy groups in a molecule and having from 2 to 10 carbon atoms constituting a side chain among carbon atoms of the constituent unit; and
(ii) a hydrocarbon group having 8 or more carbon atoms.

(2) The lipid nanoparticle according to (1), wherein a nucleic acid is encapsulated in the lipid nanoparticle.
(3) The lipid nanoparticle according to (1) or (2),
wherein the constituent unit (A) is a constituent unit represented by Chemical Formula (1) shown below:

[Chem. 1]

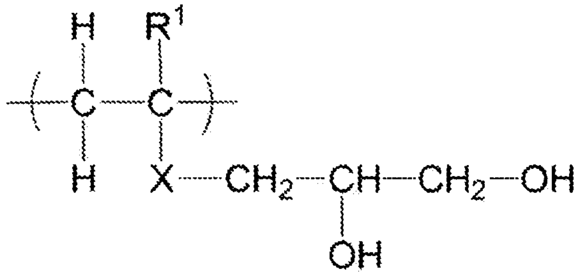

(1)

where

$R^1$ represents a hydrogen atom or a methyl group,
X represents -C(=O)-O-, -C(=O)-NH-, -O-, $-CH_2O-$, or $-CH_2CH_2O-$.

(4) The lipid nanoparticle according to (3), wherein the constituent unit (A) has a proportion in the site (I) of from 80 to 100 mass%.
(5) The lipid nanoparticle according to any one of (1) to (4), wherein the amphiphilic compound has a modification rate (%) of 0.01% or more and less than 40%, the modification rate being calculated by a formula shown below.

Modification rate (%) = {the amphiphilic compound (mol)/sum of lipids contained in the lipid nanoparticle (mol)} × 100 (%).

(6) The lipid nanoparticle according to any one of (1) to (5), further containing a cationic lipid.
(7) The lipid nanoparticle according to (6), wherein the cationic lipid has a mole ratio to a total lipid present in the lipid nanoparticle of from 0.5 to 75 mol%.
(8) The lipid nanoparticle according to any one of (1) to (7), further containing a phospholipid.
(9) The lipid nanoparticle according to (8), further containing a sterol,
wherein the phospholipid and the sterol have a content mole ratio of phospholipid : sterol = 1 : 0.1 to 1 : 40.
(10) The lipid nanoparticle according to any one of (1) to (9), wherein a particle size of the lipid nanoparticle is from 10 to 300 nm.
(11) A pharmaceutical containing the lipid nanoparticle described in any one of (1) to (10).
(12) A method for producing a lipid nanoparticle, the lipid nanoparticle containing a phospholipid, a sterol, a cationic lipid, and an amphiphilic compound,
the amphiphilic compound including:

(i) a site (I) containing a constituent unit (A) derived from a monomer (a) having two or more hydroxy groups in a molecule and having from 2 to 10 carbon atoms constituting a side chain among carbon atoms of the constituent unit; and
(ii) a hydrocarbon group having 8 or more carbon atoms,

the method including:

mixing the phospholipid, the sterol, the cationic lipid, and the amphiphilic compound with a solvent; and bringing the resulting mixture into contact with a payload to produce a lipid nanoparticle encapsulating the payload.

(13) The method for producing a lipid nanoparticle according to (12), wherein the payload is a nucleic acid.
(14) The method for producing a lipid nanoparticle according to (12) or (13),
wherein the constituent unit (A) is a constituent unit represented by Chemical Formula (1) shown below:

[Chem. 2]

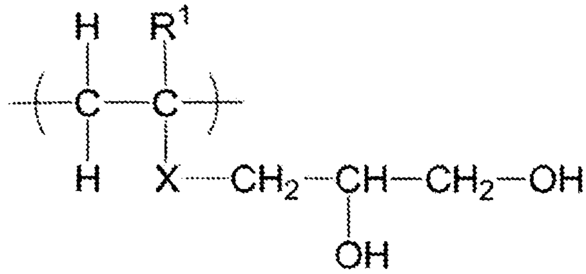

(1)

where

$R^1$ represents a hydrogen atom or a methyl group,
X represents -C(=O)-O-, -C(=O)-NH-, -O-, $-CH_2O-$, or $-CH_2CH_2O-$.

(15) The method for producing a lipid nanoparticle according to (14), wherein the constituent unit (A) has a proportion in the site (I) of from 80 to 100 mass%.

(16) The method for producing a lipid nanoparticle according to any one of (12) to (15), wherein the cationic lipid has a mole ratio to a total lipid present in the lipid nanoparticle of from 0.5 to 75 mol%.

(17) The method for producing a lipid nanoparticle according to any one of (12) to (16), wherein the phospholipid and the sterol have a content mole ratio of phospholipid : sterol = 1 : 0.1 to 1 : 40.

Brief Description of Drawings

Fig. 1 is a graph illustrating results of cell introduction of lipid nanoparticles encapsulating mRNA.

Fig. 2 is a graph illustrating results of cell introduction of lipid nanoparticles encapsulating siRNA.

Fig. 3 is a graph illustrating results of encapsulation efficiency of siRNA into lipid nanoparticles.

Fig. 4 is a graph illustrating nucleic acid recovery rates in Examples 21 to 42.

Fig. 5 is a graph illustrating relative amounts of luminescence and particle sizes of Examples 21 to 28 with respect to Comparative Example 15.

Fig. 6 is a graph illustrating relative amounts of luminescence of Examples 21 to 28 with respect to Comparative Example 15 under conditions of different pH.

Fig. 7 is a graph illustrating relative amounts of luminescence and particle sizes of Example 24 and Examples 29 to 36 with respect to Comparative Example 15.

Fig. 8 is a graph illustrating relative amounts of luminescence and particle sizes of Example 24 and Examples 37 to 42 with respect to Comparative Example 15.

Fig. 9 is a graph illustrating relative amounts of luminescence of Example 24 with respect to Comparative Example 15 in different cell types.

Description of Embodiments

One embodiment of the present invention is a lipid nanoparticle containing an amphiphilic compound. The amphiphilic compound includes (I) a site (I) containing a constituent unit (A) derived from a monomer (a) having two or more hydroxy groups in a molecule and having from 2 to 10 carbon atoms constituting a side chain among carbon atoms of the constituent unit, and (ii) a hydrocarbon group having 8 or more carbon atoms.

According to the present embodiment, the intake efficiency of the payload in the lipid nanoparticle into the cell can improve. As described above, modification is required in lipid nanoparticles, but when the lipid nanoparticles are modified with PEG, the delivery efficiency into cells is reduced as compared with the case of no modification (for example, comparison between Comparative Example 15 and Comparative Example 8 in Fig. 1 described later). However, the lipid nanoparticle modified with an amphiphilic compound of the present embodiment has improved delivery efficiency into cells as compared with the case of no modification (for example, comparison between Comparative Example 15 and Example 8 in Fig. 1 described later). Thus, the lipid nanoparticle of the present embodiment is very useful, for example, as a carrier of a payload required to be delivered into a cell for expressing a medicinal effect, such as a nucleic acid drug. In addition, when the lipid nanoparticle contains a cationic lipid, the lipid nanoparticle taken into a cell can easily escape from the endosome, and the lipid nanoparticle is easily transported into the cytoplasm. Further, according to the lipid nanoparticle of the present embodiment, good storage stability is also achieved.

Although the mechanism by which the lipid nanoparticle of the present embodiment has such excellent intracellular delivery properties is unknown, it is likely that when the amphiphilic compound of the present embodiment and each lipid (phospholipids, sterols, and cationic lipids) form a lipid nanoparticle, the lipid nanoparticle has a structure that is easily taken into cells, and as a result, the expression of drug efficacy of the payload improves.

Hereinafter, preferred embodiments of the present invention will be described. The present invention is not limited to the following embodiments. Further, a combination of two or more of the preferred embodiments of the present invention described below is also a preferred embodiment of the present invention.

In the present specification, "(from) X to Y" indicating a range means "X or more and Y or less", and "weight" and "mass" are treated as synonyms. In the present specification, "(meth)acrylate" means acrylate or methacrylate, "(meth) acryl" means acryl or methacryl, and either acrylate or methacrylate may be used alone or both of acrylate and methacrylate may be used in combination. Unless otherwise specified, operations and measurements of physical properties and the like are performed under the conditions of room temperature (20 to 25°C) and relative humidity 40 to 50%RH.

## Lipid Nanoparticle

The lipid nanoparticle of the present embodiment contains an amphiphilic compound. Further, the lipid nanoparticle of the present embodiment usually contains a phospholipid as a component constituting the lipid nanoparticle. The lipid nanoparticle of the present embodiment preferably contains a cationic lipid. When the cationic lipid is contained and the payload is anionic, the cationic lipid and the payload form a complex (aggregate), and the payload is easily packed in the lipid nanoparticle. In addition, when the cationic lipid is contained, the lipid nanoparticle can efficiently migrate from the endosome into the cytoplasm, and the function (medicinal effect) of the payload (in particular, a nucleic acid) is easily exhibited.
The particle size of the lipid nanoparticle is more than 0 nm and 1000 nm or less. In the present specification, the "particle size" means an average particle size measured by a dynamic light scattering method. The particle diameter can be measured by a particle size measuring device using a dynamic light scattering method. From the viewpoint of improving the intake into cells, the ease of sterile filtration, and the storage stability, the particle size of the lipid nanoparticle is preferably 10 nm or more, more preferably from 10 to 300 nm, still more preferably from 30 to 250 nm, yet still more preferably from 30 to 200 nm, particularly preferably from 30 to 180 nm.
Hereinafter, each component constituting the lipid nanoparticle will be described.

## Amphiphilic Compound

From the viewpoint of suppressing aggregation of lipid nanoparticles in an aqueous solvent and promoting uniform intake into cells, the number-average molecular weight (Mn) of the amphiphilic compound is preferably 1000 or more, more preferably 2000 or more, still more preferably 3000 or more. From the viewpoint of extracorporeal excretability and the like, the number-average molecular weight (Mn) of the amphiphilic compound is preferably 90000 or less, more preferably 30000 or less, still more preferably 15000 or less. The number-average molecular weight (Mn) of the amphiphilic compound is preferably from 1000 to 90000, more preferably from 2000 to 30000, still more preferably from 3000 to 15000. The value of the number-average molecular weight (Mn) can be determined by a measurement method described in Examples described later.
The amphiphilic compound according to the present embodiment includes a site (I) containing a constituent unit (A) having a predetermined structure, and a hydrocarbon group having 8 or more carbon atoms. Hereinafter, such amphiphilic compound is also simply referred to as "amphiphilic compound".
Hereinafter, these components will be described in detail.

## Site (I)

The amphiphilic compound according to the present invention first includes a site (I) containing a constituent unit (A) derived from a monomer (a) having two or more hydroxy groups in the molecule and having from 2 to 10 carbon atoms constituting a side chain among the carbon atoms of the constituent unit. In a preferred embodiment, the site (I) is made of a polymer having a constituent unit (A) derived from the monomer (a) having two or more hydroxy groups in the molecule and having from 2 to 10 carbon atoms constituting a side chain among the carbon atoms of the constituent unit. Such polymer is hereinafter also referred to as "polymer constituting the site (I)". Here, in the present specification, the "structural unit (Q) derived from a monomer (P)" (P represents any appropriate symbol, and there is a case where the notation of (P) is not present) typically means a unit (Q) (Q represents any appropriate symbol, and there is a case where the notation of (Q) is not present) constituting at least a part of a polymer, which is produced by opening one of the bonds of the polymerizable unsaturated double bond of the "monomer (P)" (or simply "monomer") through polymerization. The "structural unit derived from a monomer (P)" may be a structural unit formed by another production method as long as it has the same structure as the structural unit formed through polymerization of the monomer (P) (or simply "monomer") as described above (in the specific examples shown below, the structural unit represented by General Formula (Q)). For example, the constituent unit (A) may be formed by subjecting a structural unit formed by polymerizing a monomer (a) having a protected hydroxy group to a deprotection treatment. For example, (2-methyl-2-ethyl-1,3-dioxolan-4-yl)methyl (meth)acrylate or glycidyl ether (meth)acrylate may be polymerized and then hydrolyzed to form a structural unit derived from glycerol (meth)acrylate.
The monomer (a) having two or more hydroxy groups in the molecule and having from 2 to 10 carbon atoms constituting a side chain among the carbon atoms of the constituent unit is preferably a vinyl monomer, more preferably a (meth)acrylic monomer. The monomer (a) may be a monofunctional monomer or a polyfunctional monomer, but preferably contains a monofunctional monomer, more preferably consists of a monofunctional monomer.
Here, examples of the (meth)acrylic monomer suitably used as the monomer (a) include (meth)acrylates such as glycerol mono(meth)acrylate (also known as 2,3-dihydroxypropyl (meth)acrylate), 1,2-dihydroxyethyl (meth)acrylate, 2,2-dihydroxyethyl (meth)acrylate, dihydroxybutyl (meth)acrylate, trimethylolpropane mono(meth)acrylate, pentaerythritol mono(meth)acrylate, and dipentaerythritol mono(meth)acrylate. Among these, from the viewpoint of industrial availability

and high reactivity, glycerol monoacrylate (GLMA) and/or glycerol monomethacrylate (GLMMA) are preferable. By polymerizing these (meth)acrylic monomers (a), the ethylenic double bond contained in the monomer (a) is cleaved to generate the constituent unit (A). For the monomer (a), only one type may be used alone, or two or more types may be used in combination.

The number of carbon atoms constituting the side chain among the carbon atoms of the constituent unit (A) derived from the monomer (a) is from 2 to 10. In the present specification, the "side chain" is a portion other than the main chain. The expression "the number of carbon atoms constituting the side chain is from 2 to 10" refers to the (total) number of carbon atoms in the entire side chain (four side chains, the entire groups bound to carbon atoms in the main chain). The term "main chain" means a chain having the largest number of carbon atoms among chains of carbon atoms continuously bound in the polymer constituted by linking constituent units. As described above, the number of carbon atoms constituting the side chain among the carbon atoms of the constituent unit derived from the monomer (a) is from 2 to 10, but the number of carbon atoms is preferably from 3 to 8, more preferably from 4 to 6. The side chain of the constituent unit derived from the monomer (a) may be an unsubstituted or substituted alkyl group having from 2 to 10 carbon atoms, the substituent may be a hydroxy group, and two or more hydroxy groups as the substituents may be contained.

Here, the constituent unit (A) preferably includes a constituent unit represented by the following Chemical Formula (1).

[Chem. 3]

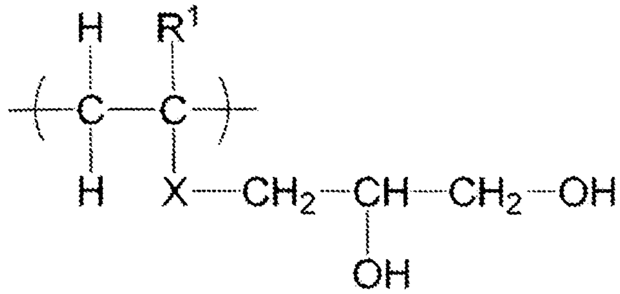

(1)

In Formula (1), $R^1$ represents a hydrogen atom or a methyl group, preferably a methyl group. X represents -C(=O)-O-, -C(=O)-NH-, -O-, $-CH_2O-$, or $-CH_2CH_2O-$, and is preferably - C(=O)-O-.

Among the constituent units represented by Chemical Formula (1), those in which $R^1$ is a hydrogen atom and X is -C(=O)-O- are derived from glycerol monoacrylate (GLMA) as the monomer (a). Among the constituent units represented by Chemical Formula (1), those in which $R^1$ is a methyl group and X is -C(=O)-O- are derived from glycerol monomethacrylate (GLMMA) as the monomer (a).

The proportion of the constituent unit (A) derived from the monomer (a) having two or more hydroxy groups in the molecule and having from 2 to 10 carbon atoms constituting the side chain among the carbon atoms of the constituent unit in the site (I) constituting the amphiphilic compound is, for example, from 1 to 100 mass%, preferably from 20 to 100 mass%, more preferably from 50 to 100 mass%, still more preferably from 60 to 100 mass%, yet still more preferably from 80 to 100 mass%, particularly preferably from 90 to 100 mass%, most preferably 100 mass%. When the proportion of the constituent unit (A) is within the range described above, the effects of the present invention can be exhibited.

When the site (I) constituting the amphiphilic compound contains a constituent unit other than the constituent unit (A) (hereinafter, also simply referred to as "constituent unit (B)"), the constituent unit (B) may be derived from any radical polymerizable monomer (hereinafter, the monomer to be the constituent unit (B) through copolymerization is also referred to as "monomer (b)"). When the site (I) constituting the amphiphilic compound contains the constituent unit (B), the proportion of the constituent unit (B) in the site (I) is, for example, 99 mass% or less, preferably 80 mass% or less, more preferably 50 mass% or less, still more preferably 40 mass% or less, yet still more preferably 20 mass% or less, particularly preferably 10 mass% or less. The site (I) constituting the amphiphilic compound does not have to contain the constituent unit (B).

Examples of the monomer (b) include a hydroxy group-containing (meth)acrylate, a polyoxyalkylene group-containing unsaturated monomer, an alkoxyalkyl (meth)acrylate, a vinyl monomer, and a cyclic compound, other than the monomer (a). For these monomers (b), only one type may be used alone, or two or more types may be used in combination.

Examples of the hydroxy group-containing (meth)acrylate include hydroxyalkyl (meth)acrylates having a hydroxyalkyl group with 2 to 4 carbon atoms, such as 2-hydroxyethyl acrylate and 2-hydroxyethyl methacrylate.

Examples of the polyoxyalkylene group-containing unsaturated monomer include a monomer represented by the following Chemical Formula (2).

[Chem. 4]

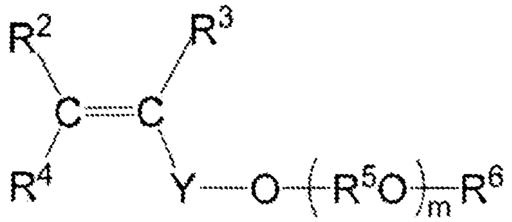

(2)

In Formula (2), $R^2$, $R^3$, and $R^4$ each independently represent a hydrogen atom or a methyl group, $R^5$ represents an alkylene group having from 2 to 18 carbon atoms, $R^6$ represents a hydrogen atom or a hydrocarbon group having from 1 to 20 carbon atoms (preferably an alkyl group or an alkenyl group), Y represents an alkylene group having from 1 to 5 carbon atoms, or a -CO- group, or represents a direct bond when the $R^2R^4C=CR^3$- group is a vinyl group, and m represents the average number of moles of addition (average value of number of moles of oxyalkylene group per mol of polyoxyalkylene group-containing unsaturated monomer) of the - $(R^5O)$- group and represents the number of from 1 to 300. In Formula (2), when $(R^5O)_m$ is composed of two or more types of $R^5O$, the two or more types of $R^5O$ may be in any combination form of random, block, and alternate.

Examples of the polyoxyalkylene group-containing unsaturated monomer include an unsaturated alcohol polyalkylene glycol adduct, a polyalkylene glycol ester-based monomer, and an (alkoxy)polyalkylene glycol monomaleic acid ester.

The unsaturated alcohol polyalkylene glycol adduct is a compound produced by adding a polyalkylene glycol chain to an alcohol having an unsaturated group. Examples of the unsaturated alcohol polyalkylene glycol adduct include polyethylene glycol monovinyl ether, polyethylene glycol monoallyl ether, polyethylene glycol mono(2-methyl-2-propenyl) ether, polyethylene glycol mono(2-butenyl) ether, polyethylene glycol mono(3-methyl-3-butenyl) ether, polyethylene glycol mono(3-methyl-2-butenyl) ether, polyethylene glycol mono(2-methyl-3-butenyl) ether, polyethylene glycol mono(2-methyl-2-butenyl) ether, polyethylene glycol mono(1,1-dimethyl-2-propenyl) ether, polyethylene polypropylene glycol mono(3-methyl-3-butenyl) ether, and methoxy polyethylene glycol mono(3-methyl-3-butenyl) ether.

The polyalkylene glycol ester-based monomer is a monomer produced by binding an unsaturated group and a polyalkylene glycol chain via an ester bond.

As the polyalkylene glycol ester-based monomer, for example, an esterified product of (meth)acrylic acid and an alkoxypolyalkylene glycol produced by adding from 1 to 300 mol of an oxyalkylene group having from 2 to 18 carbon atoms to an alcohol is preferable. Among the alkoxy polyalkylene glycols, those having an oxyethylene group as a main component are preferable. Examples of the alcohol include aliphatic alcohols having from 1 to 30 carbon atoms such as methanol, ethanol, 1-propanol, 2-propanol, 1-butanol, 2-butanol, 1-pentanol, 2-pentanol, 3-pentanol, 1-hexanol, 2-hexanol, 3-hexanol, octanol, 2-ethyl-1-hexanol, nonyl alcohol, lauryl alcohol, cetyl alcohol, and stearyl alcohol; alicyclic alcohols having from 3 to 30 carbon atoms such as cyclohexanol; and unsaturated alcohols having from 3 to 30 carbon atoms such as (meth)allyl alcohol, 3-butene-1-ol, and 3-methyl-3-butene-1-ol. Examples of the esterified product include methoxy polyethylene glycol mono(meth)acrylate, methoxy (polyethylene glycol polypropylene glycol) mono(meth)acrylate, methoxy (polyethylene glycol polybutylene glycol) mono(meth)acrylate, and methoxy (polyethylene glycol polypropylene glycol polybutylene glycol) mono(meth)acrylate. Among the polyalkylene glycol ester-based monomers, for example, (alkoxy) polyalkylene glycol mono (meth)acrylates such as methoxypolyethylene glycol monomethacrylate are preferable.

Examples of the alkoxyalkyl (meth)acrylate include alkoxyalkyl (meth)acrylates in which an alkoxy group has from 1 to 4 carbon atoms and an alkyl group has from 1 to 4 carbon atoms, such as methoxymethyl (meth)acrylate, methoxyethyl (meth)acrylate, methoxypropyl (meth)acrylate, ethoxymethyl (meth)acrylate, ethoxyethyl (meth)acrylate, and ethoxypropyl (meth)acrylate. For these alkoxyalkyl (meth)acrylates, a single type may be used alone or two or more types may be used in combination.

Examples of the vinyl monomer include (meth)acrylic acid, methyl (meth)acrylate, ethyl (meth)acrylate, propyl (meth)acrylate, n-butyl (meth)acrylate, isobutyl (meth)acrylate, tert-butyl (meth)acrylate, 2-ethylhexyl (meth)acrylate, n-lauryl (meth)acrylate, n-stearyl (meth)acrylate, diaminomethyl (meth)acrylate, diaminoethyl (meth)acrylate, dimethylamino (meth)acrylate, diethylamino (meth)acrylate, glycidyl (meth)acrylate, styrene, aziridines, 2-(meth)acryloyloxymethylphosphorylcholine, 2-(meth)acryloyloxyethylphosphorylcholine, tetrahydrofuryl (meth)acrylate, isopropylacrylamide, vinyl alcohol, vinylformamide, vinylisobutylacrylamide, (meth)acrylamide, dimethylacrylamide, vinylacetamide, and N-vinylpyrrolidone.

Examples of the cyclic compound include lactides such as L-lactide, lactones such as ε-caprolactone, trimethyl carbonate, cyclic amino acids, and morpholine-2,5-dione.

When the site (I) is made of a polymer including the constituent unit (A) (hereinafter, it is also simply referred to as

such polymer or the polymer), such polymer may have a configuration of a block copolymer produced by bonding polymers of the same type or different types.

From the viewpoint of, for example, improving the expression level of nucleic acid through uniform dispersion of particles, the number-average molecular weight (Mn) of the polymer is preferably 1000 or more, more preferably 2000 or more, still more preferably 3000 or more. From the viewpoint of extracorporeal excretability and the like, the number-average molecular weight (Mn) of the polymer is preferably 90000 or less, more preferably 30000 or less, still more preferably 15000 or less. The number-average molecular weight (Mn) of the polymer may be from 1000 to 90000, from 2000 to 30000, or from 3000 to 15000. Here, in Examples described later, because the number-average molecular weight (Mn) of the amphiphilic compound is measured, the value of Mn of the site (I) (polymer) can be calculated by subtracting the molecular weight of the site other than the site (I) (polymer) from the measured value. When an accurate value can be calculated, the value of Mn may be obtained by the same method using only the site (I) (polymer).

From the viewpoint of, for example, the modifying property of the amphiphilic compound (polymer) to lipid nanoparticles, the molecular weight distribution (value of [polymerization-average molecular weight (Mw)/number-average molecular weight (Mn)]) of the polymer is preferably from 1 to 5, more preferably from 1 to 3, still more preferably from 1 to 2, still more preferably from 1 to 1.5, still more preferably from 1 to 1.3.

The polymer constituting the site (I) can be produced by polymerizing a monomer composition containing the monomer (a) and, as necessary, the monomer (b). Examples of the method for polymerizing the monomer composition include a living radical polymerization method represented by a radical polymerization method, an atom transfer radical polymerization method, a reversible addition-fragmentation chain transfer (RAFT) polymerization method, and the like, an ion polymerization method, a ring-opening polymerization method, a coordination polymerization method, a polycondensation method, and the like, but the present invention is not limited to these examples.

When the monomer composition is polymerized, a solvent may be used. Examples of the solvent include aromatic solvents such as benzene, toluene, and xylene; alcohol-based solvents such as methanol, ethanol, isopropanol, n-butanol, and tert-butanol; halogen atom-containing solvents such as dichloroethane and dichloromethane; ether-based solvents such as diethyl ether, propylene glycol methyl ether, dipropylene glycol methyl ether, ethyl cellosolve, and butyl cellosolve; ester-based solvents such as ethyl acetate, butyl acetate, and cellosolve acetate; ketone-based solvents such as acetone, methyl ethyl ketone, methyl isobutyl ketone, and diacetone alcohol; organic solvents such as amide-based solvents such as dimethylformamide; and water. Among these, from the viewpoint of reactivity, an alcohol-based solvent and/or water is preferably used. For these solvents, a single type may be used alone or two or more types may be used in combination. The amount of the solvent may be appropriately determined in consideration of polymerization conditions, the composition of the monomer composition, the concentration of the resulting polymer, and the like.

When the monomer composition is polymerized, a chain transfer agent can be used to adjust the molecular weight of the polymer or to introduce a hydrocarbon group (including a hydrocarbon group having 8 or more carbon atoms described later).

The amount of the chain transfer agent may be appropriately set according to the type of the monomer contained in the monomer composition, the polymerization conditions such as a polymerization temperature, the molecular weight of a target polymer, and the like, and is not particularly limited.

When the monomer composition is polymerized, a polymerization initiator can be used.

Examples of the polymerization initiator include a radical polymerization initiator and a living radical polymerization initiator. For these polymerization initiators, a single type may be used alone or two or more types may be used in combination.

The amount of the polymerization initiator may be appropriately set according to desired physical properties and the like of the resulting polymer.

The polymerization conditions for polymerizing the monomer composition may be appropriately set according to the polymerization method, and are not particularly limited.

The polymer constituting the site (I) can be produced by polymerizing the monomer composition as described above. Here, the resulting polymer may have a functional group at its terminal. When the polymer constituting the site (I) has a functional group at its terminal, a pharmaceutical or the like can be easily modified via the functional group, or the polymer can be easily linked to a predetermined hydrocarbon group described later via the functional group. However, the polymer constituting the site (I) does not have to have a functional group at its terminal. The amphiphilic compound according to the present invention has a hydrocarbon group having 8 or more carbon atoms and the site (I) (including a polymer) in the molecule, and when the polymer constituting the site (I) has a functional group at its terminal, the functional group may be present at only one terminal of the polymer or may be present at both terminals. The functional group present at a terminal of the polymer may be positioned on the side where the site (I) is bound to a predetermined hydrocarbon group described later, or may be positioned on the opposite side.

As the functional group that can be possessed by the polymer constituting the site (I), an anionic functional group, a cationic functional group, a nonionic functional group, and an amphoteric functional group are preferable. The functional group is preferably a reactive functional group. Examples of suitable reactive functional groups include -SH group, a group

represented by Formula: -COOM (where M represents a hydrogen atom or an alkali metal atom), hydroxy group, allyl group, epoxy group, aldehyde group, $-NH_2$ group, and CONH- group. Examples of M include alkali metal atoms such as a sodium atom and a potassium atom. When the polymer has a functional group at its terminal, the number of the functional group is not particularly limited, but is preferably from 1 to 6, more preferably from 1 to 4, still more preferably from 1 to 2.

To introduce a functional group to a terminal of the polymer constituting the site (I), a functional group-containing compound for introducing the functional group into the polymer can be used. Examples of the functional group-containing compound for introducing the functional group to a terminal of the polymer include thiol-based chain transfer agents such as alkali metal thioacetates, for example, sodium thioacetate and potassium thioacetate, cysteine, cysteamine, mercaptoethanol, thioglycerol, thioglycolic acid, mercaptopropionic acid, 2-mercaptopropionic acid, 3-mercaptopropionic acid, thioacetic acid, thiomalic acid, 2-mercaptoethanesulfonic acid, and sodium salts and potassium salts thereof; RAFT agents such as 4-cyano-4-(phenylcarbonothioylthio) pentanoic acid; and a polymerization initiator into which a functional group such as 4,4'-azobis(4-cyanopentanoic acid), 2,2' -azobis[2-methyl-N-[1,1-bis(hydroxymethyl)-2-hydroxyethyl] propionamide], 2,2' -azobis[N-(2-hydroxyethyl)-2-methoxypropanamido], 2,2'-azobis(2-methyl-2-propenylpropanamido), 2,2'-bis(2-imidazolin-2-yl)[2,2'-azobispropane]dihydrochloride, 2,2'-azobis(propane-2-carboamidine)dihydrochloride, 2,2'-azobis[N-(2-carboxyethyl)-2-methylpropionamidine], 2,2'-azobis[2-[1-(2-hydroxyethyl)-2-imidazolin-2-yl]propane dihydrochloride, cyclohexanone peroxide, or acetylacetone peroxide is introduced. For these functional group-containing compounds, a single type may be used alone or two or more types may be used in combination. Among the functional group-containing compounds described above, those corresponding to the chain transfer agent or the polymerization initiator described above are included. The functional group-containing compound corresponding to the chain transfer agent or the polymerization initiator may be used only either for the chain transfer agent or the polymerization initiator or for the functional group-containing compound, or may be used for both purposes.

When a living polymerization initiator is used as the polymerization initiator, a functional group-containing compound may be reacted with a halogen atom present at a terminal of a polymer prepared using the living polymerization initiator to introduce a functional group to the terminal of the polymer. Examples of such a functional group-containing compound capable of reacting with a halogen atom to introduce a functional group to a terminal of the polymer include amine compounds such as ethylenediamine and propyldiamine, dithiol compounds such as ethanedithiol, propanedithiol, and hexadecanedithiol, and thiol compounds such as allyl mercaptan, cysteine, cysteamine, mercaptoethanol, thioglycerol, thioglycolic acid, mercaptopropionic acid, 2-mercaptopropionic acid, 3-mercaptopropionic acid, thioacetic acid, thiomalic acid, 2-mercaptoethanesulfonic acid, and sodium salts and potassium salts thereof.

The amount of the functional group-containing compound for introducing a functional group to a terminal of the polymer may be appropriately set according to the type of the monomer (constituent unit) constituting the polymer, polymerization conditions such as a polymerization temperature, the molecular weight of a target polymer, and the like, and is not particularly limited. When a polymer having a number-average molecular weight of several thousands to several tens of thousands is produced, the amount of the chain transfer agent per 100 parts by mass of the monomer is preferably from 0.1 to 20 parts by mass, more preferably from 0.5 to 15 parts by mass.

Examples of the method for introducing a functional group into a terminal of the polymer constituting the site (I) include:

(1) a method of polymerizing a monomer composition in the presence of a polymerization initiator having the functional group introduced as a polymerization initiator to produce a polymer;
(2) a method of polymerizing a monomer composition in the presence of a chain transfer agent having the functional group introduced as a chain transfer agent to produce a polymer; and
(3) a method of reacting the halogen atom present at a terminal of the polymer with a functional group-containing compound.

The present invention is not limited only to such examples.

Hydrocarbon Group

The amphiphilic compound according to the present invention is characterized in that it also has a hydrocarbon group having 8 or more carbon atoms in addition to the above-described "site (I) containing the constituent unit (A) derived from the monomer (a) having two or more hydroxy groups in the molecule". The specific configuration of the hydrocarbon group is not particularly limited.

As an example, the hydrocarbon group is preferably a group contained in an organic compound having performance of forming an aggregate of molecules through hydrophobic interaction in an aqueous solution. Examples of such an organic compound include hydrocarbons, hydrophobic polymers, lipids, and other organic molecules. Preferably, the organic compound is a hydrocarbon and/or a lipid, more preferably a hydrocarbon.

Examples of the hydrocarbon include aliphatic hydrocarbons having from 8 to 50 carbon atoms and aromatic

hydrocarbons having from 8 to 50 carbon atoms. That is, in a preferred embodiment of the present invention, the number of carbon atoms of the hydrocarbon group is from 8 to 50, more preferably from 8 to 40, still more preferably from 8 to 30, particularly preferably from 8 to 20. Preferably, the hydrocarbon is an aliphatic hydrocarbon having from 8 to 50 carbon atoms (the hydrocarbon group is an aliphatic hydrocarbon group having from 8 to 50 carbon atoms.).

Examples of the aliphatic hydrocarbon having from 8 to 50 carbon atoms include linear alkanes such as octane, nonane, decane, undecane, dodecane, tridecane, tetradecane, pentadecane, hexadecane, heptadecane, octadecane, nonadecane, and icosane, branched alkanes of these, and cyclic alkanes of these. Linear alkanes are preferable.

Examples of the aromatic hydrocarbon having from 8 to 50 carbon atoms include 2-phenylethane, 1,3,5-trimethylbenzene, naphthalene, anthracene, fluorescein, and positional isomers of these.

Examples of the hydrophobic polymer include a polymer produced by polymerizing, as a main component, a vinyl monomer having a hydrocarbon group having 8 or more carbon atoms in a side chain among the vinyl monomers that can serve as the constituent unit of the site (I) described above.

The "lipid" means an organic compound that has a hydrophobic moiety (for example, long chain fatty acids, hydrocarbon chains, sterol groups), is poorly soluble in water, and is easily soluble in an organic solvent. The lipid usually has a hydrophilic group such as a hydroxy group, a carboxylic acid (carboxy group), or an amino group. In this case, the hydrophobic moiety preferably has 8 or more carbon atoms. Thus, the amphiphilic compound of the present invention is also included in the lipid. "Lipids" can be further roughly classified into phospholipids, glycolipids (glyceroglycolipids and glycosphingolipids), sphingolipids, sterols, neutral lipids, saturated or unsaturated fatty acids, and the like.

Phospholipids are roughly classified into glycerophospholipids and sphingophospholipids. Representative examples of the glycerophospholipids include phosphatidylcholine (PC), phosphatidylserine (PS), phosphatidylinositol (PI), phosphatidylglycerol (PG), phosphatidylethanolamine (PE), and phosphatidic acid (PA). On the other hand, a representative example of the sphingophospholipids is sphingomyelin. Specific examples of the phospholipids include the lipids described in the following (a) to (g).

(a) Phosphatidylcholines

Specific examples of the phosphatidylcholines include dipalmitoylphosphatidylcholine (DPPC), distearoylphosphatidylcholine (DSPC), dimyristoylphosphatidylcholine (DMPC), dioleoylphosphatidylcholine (DOPC), dilauroylphosphatidylcholine (DLPC), didecanoylphosphatidylcholine (DDPC), dioctanoylphosphatidylcholine, dihexanoylphosphatidylcholine (DHPC), dibutyrylphosphatidylcholine (DBPC), dielaidoylphosphatidylcholine, dilinoleoylphosphatidylcholine, diarachidonoylphosphatidylcholine, dieicosenoylphosphatidylcholine (DEPC), diheptanoylphosphatidylcholine, dicaproylphosphatidylcholine, diheptadecanoylphosphatidylcholine, dibehenoylphosphatidylcholine, eleostearoylphosphatidylcholine, hydrogenated egg phosphatidylcholine (HEPC), hydrogenated soybean phosphatidylcholine (HSPC), 1-palmitoyl-2-arachidonoylphosphatidylcholine, 1-palmitoyl-2-oleoylphosphatidylcholine, 1-palmitoyl-2-linoleoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, 1,2-dimyristoylamide-1,2-deoxyphosphatidylcholine, 1-myristoyl-2-palmitoylphosphatidylcholine, 1-myristoyl-2-stearoylphosphatidylcholine, di-0-hexadecylphosphatidylcholine, trans-dielaidoylphosphatidylcholine, dipalmitotetraidoylphosphatidylcholine, n-octadecyl-2-methylphosphatidylcholine, n-octadecylphosphatidylcholine, 1-laurylpropanediol-3-phosphocholine, erythro-N-lignoceroylsphingophosphatidylcholine, and palmitoyl-(9-cis-octadecenoyl)-3-sn-phosphatidylcholine.

(b) Phosphatidylserines

Specific examples of the phosphatidylserines include distearoylphosphatidylserine (DSPS), dimyristoylphosphatidylserine (DMPS), dilauroylphosphatidylserine (DLPS), dipalmitoylphosphatidylserine (DPPS), dioleoylphosphatidylserine (DOPS), lysophosphatidylserine, eleostearoylphosphatidylserine, and 1,2-di-(9-cis-octadecenoyl)-3-sn-phosphatidylserine.

(c) Phosphatidylinositols

Specific examples of the phosphatidylinositols include dipalmitoylphosphatidylinositol (DPPI), distearoylphosphatidylinositol (DSPI), and dilauroylphosphatidylinositol (DLPI).

(d) Phosphatidylglycerols

Specific examples of the phosphatidylglycerols include dipalmitoylphosphatidylglycerol (DPPG), distearoylphosphatidylglycerol (DSPG), dioleoylphosphatidylglycerol (DOPG), dilauroylphosphatidylglycerol (DLPG), dimyristoylphosphatidylglycerol (DMPG), lysophosphatidylglycerol, hydrogenated soy phosphatidylglycerol (HSPG), hydrogenated egg

phosphatidylglycerol (HEPG), and cardiolipin (diphosphatidylglycerol).

(e) Phosphatidylethanolamines (cephalins)

Specific examples of the phosphatidylethanolamines (cephalins) include dipalmitoylphosphatidylethanolamine (DPPE), distearoylphosphatidylethanolamine (DSPE), dioleoylphosphatidylethanolamine (DOPE), dilauroylphosphatidylethanolamine (DLPE), dimyristoylphosphatidylethanolamine (DMPE), didecanoylphosphatidylethanolamine (DDPE), N-glutarylphosphatidylethanolamine (NGPE), lysophosphatidylethanolamine, N-(7-nitro-2,1,3-benzoxydiazole-4-yl)-1,2-dioleoyl-sn-phosphatidylethanolamine, eleosteroylphosphatidylethanolamine, N-succinyldioleoylphosphatidylethanolamine, and 1-hexadecyl-2-palmitoylglycerophosphatidylethanolamine. In Production Example 5 described later, distearoyl N-(3-maleimide-1-oxopropyl)-L-α-phosphatidylethanolamine (COATSOME (trade name) FE-8080MA3 manufactured by NOF CORPORATION), which is a distearoylphosphatidylethanolamine (DSPE) derivative having a maleimide group, is used as a raw material of lipid. The maleimide group can be bound to the polymer constituting the site (I) by reacting with a thiol group that can be introduced to a terminal of the polymer constituting the site (I). Thus, phosphatidylethanolamines (and other lipid derivatives) having not only hydrocarbon groups having various carbon atom numbers of 8 or more can also be used in the same manner but also a functional group such as a maleimide group or a succinimide group for linking to the site (I).

(f) Phosphatidic Acids

Specific examples of the phosphatidic acids include dipalmitoylphosphatidic acid (DPPA), distearoylphosphatidic acid (DSPA), dimyristoylphosphatidic acid (DMPA), and dioleylphosphatidic acid (DOPA).

(g) Sphingophospholipid

Specific examples of the sphingophospholipids include sphingomyelin, dipalmitoylsphingomyelin, distearoylsphingomyelin, ceramide ciliatine, ceramide phosphorylethanolamine, and ceramide phosphorylglycerol.

The most typical sterol is cholesterol. In addition to cholesterol, examples of the sterols include cholesterol succinic acid, dihydrocholesterol, lanosterol, dihydrolanosterol, desmosterol, stigmasterol, sitosterol, campesterol, brassicasterol, timosterol, ergosterol, campesterol, fucosterol, 22-ketosterol, 20-hydroxycholesterol, 7-hydroxycholesterol, 19-hydroxycholesterol, 22-hydroxycholesterol, 25-hydroxycholesterol, 7-dehydrocholesterol, 5α-cholest-7-en-3β-ol, epicholesterol, dehydroergosterol, sulfate cholesterol, hemisuccinate cholesterol, cholesterol phthalate, cholesterol phosphate, cholesterol valerate, cholesterol hemisuccinate, 3βN-(N',N'-dimethylaminoethane)-carbamoyl cholesterol, cholesterol acetate, cholesteryl olate, cholesteryl linolate, cholesteryl myristate, cholesteryl palmitate, cholesteryl arachidate, coprostanol, cholesterol esters, cholesteryl phosphorylcholine, and 3,6,9-trioxaoctan-1-ol-cholesteryl-3e-ol.

Examples of the neutral lipids include diglycerides (for example, diolein, dipalmitolein) and mixed caprylin-caprine diglycerides, triacylglycerols (triolein, tripalmitolein, trimyristolein, trilaurin, tricaprine, tricaprylin, tricaproin, and the like), squalene, and tocopherol.

Examples of the saturated fatty acids and unsaturated fatty acids include saturated or unsaturated fatty acids having from 5 to 30 carbon atoms.

When other organic molecules (regardless of natural compound or chemically synthesized compound) have a hydrocarbon group having 8 or more carbon atoms, they can also be used as a source of the hydrocarbon group having 8 or more carbon atoms in the amphiphilic compound according to the present invention.

Here, in a preferred embodiment of the present invention, the hydrocarbon group having 8 or more carbon atoms (preferably from 8 to 50 carbon atoms) is preferably bound to the site (I) containing the constituent unit (A) directly or via a divalent bonding group, or present as a part of a lipid (for example, a phospholipid) that is bound to the site (I) containing the constituent unit (A) directly or via a divalent bonding group.

Similarly to the polymer constituting the site (I), the hydrocarbon group having 8 or more carbon atoms may also have a functional group at its terminal. When the hydrocarbon group having 8 or more carbon atoms has a functional group at its terminal, it is possible to modify a pharmaceutical or the like through the functional group. However, the hydrocarbon group having 8 or more carbon atoms does not have to have a functional group at its terminal. When the hydrocarbon group having 8 or more carbon atoms has a functional group at its terminal, the functional group may be present only at one terminal of the hydrocarbon group having 8 or more carbon atoms or may be present at both terminals. The functional group present at a terminal of the hydrocarbon group having 8 or more carbon atoms may be positioned on the side where the hydrocarbon group having 8 or more carbon atoms is bound to the site (I), or may be positioned on the opposite side.

From the viewpoint of extracorporeal excretability and the like, the molecular weight of the hydrocarbon group having 8 or more carbon atoms is preferably from 100 to 5000, more preferably from 150 to 2000, still more preferably from 150 to 1000. Here, the proportion of the molecular weight of the "hydrocarbon group having 8 or more carbon atoms" to the

molecular weight of the amphiphilic compound is preferably from 0.2 to 20%, more preferably from 0.5 to 15%, still more preferably from 1 to 10%.

In the present invention, when the hydrocarbon group having 8 or more carbon atoms is present as a part of the lipid in the amphiphilic compound, the molecular weight of the lipid is preferably from 100 to 2000, more preferably from 150 to 1000. Here, when the hydrocarbon group having 8 or more carbon atoms is present as a part of the lipid in the amphiphilic compound, the proportion of the molecular weight of the lipid to the molecular weight of the amphiphilic compound is preferably from 2 to 50%, more preferably from 5 to 35%, still more preferably from 10 to 20%.

The divalent bonding group is a group via which the site (I) and the hydrocarbon group having 8 or more carbon atoms are bonded, and the structure thereof is not particularly limited. The divalent bonding group may be contained in a part of the structure of the site (I). Specific examples of such a divalent bonding group include -S-, -S-C(=S)-, -S-C(=S)-S-, -S-C(=S)-N(-$R_a$)-, -S-C(=S)-O-, -S-$R_b$-C(=O)-O-, -S-$R_b$-C(=O)-N(-$R_a$)-, -S-$R_b$-O-, -S-$R_b$-O-C(=O)-, -O-, -O-C(=O)-, -N(-$R_a$)-C(=O)-, and a divalent bonding group containing these. Here, $R_a$ is a hydrogen atom or a hydrocarbon group having from 1 to 30 carbon atoms. $R_b$ is a hydrocarbon group having from 1 to 30 carbon atoms. Residues produced by excluding one hydrogen atom and one hydrocarbon group having 8 or more carbon atoms from lipids (including modified products of lipids) are also one of preferred forms of the divalent bonding group. The divalent bonding group preferably has a molecular weight of 5000 or less, more preferably 2000 or less, still more preferably 1000 or less.

Here, examples of the method for linking a hydrocarbon group having 8 or more carbon atoms to the site (I) (polymer constituting the site (I)) via a divalent bonding group include a method in which when a monomer composition is polymerized in the presence of a chain transfer agent and/or a polymerization initiator to produce a polymer constituting the site (I), a chain transfer agent or a polymerization initiator containing in the molecule a structure capable of constituting a site containing a hydrocarbon group having 8 or more carbon atoms is used as the chain transfer agent and/or the polymerization initiator. In particular, by polymerizing the monomer composition using a chain transfer agent (octanethiol, decanethiol, dodecanethiol, hexadecanethiol, octadecanethiol, and the like) having a thiol group (-SH group) bound to a hydrocarbon group having 8 or more carbon atoms (the number of carbon atoms is preferably from 8 to 50, more preferably from 8 to 40, still more preferably from 8 to 30, particularly preferably from 8 to 20), it is possible to produce an amphiphilic compound having the site (I) bound to a hydrocarbon group having 8 or more carbon atoms via a thioether bond (-S-) (see Production Examples 1 to 4 described later). In a preferred embodiment of the present invention, the amphiphilic compound is produced by polymerizing a monomer composition containing a chain transfer agent containing a vinyl monomer (preferably, a (meth)acrylic monomer, more preferably glycerol mono (meth)acrylate) and a thiol group (-SH group) bound to a hydrocarbon group having 8 or more carbon atoms.

As another method for linking a hydrocarbon group having 8 or more carbon atoms to the site (I) (polymer constituting the site (I)) via a divalent bonding group, first, when a monomer composition is polymerized in the presence of a chain transfer agent to produce a polymer constituting the site (I), reversible addition-fragmentation chain transfer (RAFT) polymerization is performed using an RAFT agent as the chain transfer agent, thereby introducing a thiol group (-SH group) at a terminal of the polymer constituting the site (I). Subsequently, a compound having not only the hydrocarbon group having 8 or more carbon atoms but also a functional group (for example, a maleimide group or the like) capable of reacting with a thiol group (-SH group) is reacted with the introduced thiol group (-SH group). This makes it possible to link the hydrocarbon group having 8 or more carbon atoms and the site (I) via a thioether bond (-S-) (see Production Example 5 described later). This approach is particularly useful when the hydrocarbon group having 8 or more carbon atoms is present in the amphiphilic compound as part of a lipid or other organic molecule.

In the amphiphilic compound according to the present invention, when the polymer constituting the site (I) or the hydrocarbon group having 8 or more carbon atoms is present in the compound as a part of the polymer, the polymer may have a crosslinked structure.

Modification Rate of Amphiphilic Compound

In one embodiment, the modification rate (% (mol)) of the lipid nanoparticle with the amphiphilic compound is 0.005% or more, 0.01% or more, 0.05% or more, 0.1% or more, 0.2% or more, 0.3% or more, 0.4% or more, 0.5% or more, 0.6% or more, 0.7% or more, 0.8% or more, or 1% or more. In one embodiment, the modification rate (%) of the lipid nanoparticle with the amphiphilic compound is less than 40%, 30% or less, in particular, 20% or less, 18% or less, 16% or less, 15% or less, 12% or less, or 10% or less. In one embodiment, the modification rate (%) of the lipid nanoparticle with the amphiphilic compound may be 5% or less (for example, from 0.005 to 5%, or from 0.01 to 5%), or less than 5% (for example, 0.005% or more and less than 5%, or 0.01% or more and less than 5%). In one embodiment, the modification rate (% (mol)) of the lipid nanoparticle with the amphiphilic compound is 0.005% or more and less than 40%, 0.01% or more and less than 40%, 0.05% or more and less than 40%, from 0.1 to 30%, from 0.1 to 20%, from 0.2 to 18%, from 0.3 to 16%, from 0.4 to 15%, from 0.5 to 12%, from 0.6 to 10%, from 0.7 to 10%, from 0.8 to 10%, from 1 to 10%, from 0.005 to 5%, or 0.005% or more and less than 5%. When the modification rate is equal to or more than the lower limit, the effect of improving the introduction of the lipid nanoparticle into a cell with the amphiphilic compound is easily achieved. When the modification

rate is equal to or more than the lower limit, the encapsulation efficiency of the payload (for example, nucleic acid) improves. In addition, the retention in blood of the lipid nanoparticles improves. When the modification rate is equal to or less than the upper limit, the particle stability of the lipid nanoparticle improves, the effect of improving introduction of the lipid nanoparticle into a cell with the amphiphilic compound is easily achieved, and the function is easily exhibited (for example, when the payload is a nucleic acid, high expression efficiency is likely to be achieved).

The modification rate of the lipid nanoparticle with the amphiphilic compound can be determined by the following formula.

Modification rate (%) = {amphiphilic compound (mol)/sum of lipids contained in lipid nanoparticle (mol)} × 100 (%).

The sum of the lipids contained in the lipid nanoparticle is, for example, the sum of phospholipids, cationic lipids, amphiphilic compounds, and sterols. Thus, in one preferred aspect of the present invention, the modification rate (A) (%) shown below is 0.01% or more and less than 40%. In one embodiment, the modification rate (A) (% (mol%)) may be any of 0.005% or more and less than 40%, 0.01% or more and less than 40%, 0.05% or more and less than 40%, from 0.1 to 30%, from 0.1 to 20%, from 0.2 to 18%, from 0.3 to 16%, from 0.4 to 15%, from 0.5 to 12%, from 0.6 to 10%, from 0.7 to 10%, from 0.8 to 10%, from 1 to 10%, from 0.005 to 5%, or 0.005% or more and less than 5%.

Modification rate (A) (%) = {amphiphilic compound (mol)/sum (mol) of phospholipid, cationic lipid, amphiphilic compound, and sterol contained in lipid nanoparticle} ×100 (%).

In the present specification, the amount of the amphiphilic compound and the total amount of the lipids in the lipid nanoparticle are matched with the amount of each lipid added during the production process, and after extraction from the lipid nanoparticle using an organic solvent (for example, ethanol or methanol) or a surfactant (for example, Triton (trade name) X100 or Tween 80), the total amount of the amphiphilic compound and the total amount of the lipids can be measured by using HPLC.

Phospholipid

Examples of the phospholipid include the lipids described in the section of the amphiphilic compound.

For the phospholipids used for the lipid nanoparticle, a single type may be used alone, or two or more types may be used in combination.

The phospholipid used for the lipid nanoparticle preferably contains phosphatidylcholines and phosphatidylethanolamines from the viewpoint of particle formability.

Specific examples of phosphatidylcholines include those listed above. For the phosphatidylcholines, a single type may be used alone, or two or more types may be used in combination.

From the viewpoint of particle formability, the phospholipid used for the lipid nanoparticle preferably contains phosphatidylcholines, more preferably contains hydrogenated egg phosphatidylcholine (HEPC), hydrogenated soybean phosphatidylcholine (HSPC), dipalmitoylphosphatidylcholine (DPPC), distearoylphosphatidylcholine (DSPC), dimyristoylphosphatidylcholine (DMPC), or a mixture thereof, still more preferably contains distearoylphosphatidylcholine (DSPC), hydrogenated egg phosphatidylcholine (HEPC), hydrogenated soybean phosphatidylcholine (HSPC), or a mixture thereof, yet still more preferably contains distearoylphosphatidylcholine (DSPC) and/or hydrogenated soybean phosphatidylcholine (HSPC), and it may be composed only of distearoylphosphatidylcholine (DSPC) and/or hydrogenated soybean phosphatidylcholine (HSPC).

It is also preferable to use a phospholipid having an unsaturated structure because the function of the payload is easily exhibited (for example, when the payload is a nucleic acid, high expression efficiency is likely to be achieved). Examples of the phospholipid having an unsaturated structure include dioleoylphosphatidylcholine (DOPC), dioleoylphosphatidylserine (DOPS), dioleoylphosphatidylglycerol (DOPG), dioleoylphosphatidylethanolamine (DOPE), and dioleylphosphatidic acid (DOPA). In one embodiment, the phospholipid used in the lipid nanoparticle is a combination of phosphatidylcholines and a phospholipid having an unsaturated structure, preferably a combination of distearoylphosphatidylcholine (DSPC), hydrogenated egg phosphatidylcholine (HEPC), hydrogenated soybean phosphatidylcholine (HSPC), or a mixture thereof, and a phospholipid having an unsaturated structure, more preferably a combination of distearoylphosphatidylcholine (DSPC) and/or hydrogenated soybean phosphatidylcholine (HSPC) and dioleoylphosphatidylethanolamine (DOPE). When phosphatidylcholines (not including phospholipids having an unsaturated structure) and a phospholipid having an unsaturated structure are used in combination, the content mass ratio is, for example, phosphatidylcholines (not including phospholipids having an unsaturated structure): phospholipid having an unsaturated structure = 1 : 0.05 to 50 (mole ratio), and it may be 1 : 0.1 to 10 (mole ratio).

The proportion (mol%) of the phospholipid to the total lipid present in the lipid nanoparticle is, for example, from 1

to 80 mol%, from 2 to 70 mol%, or from 2 to 60 mol%, and in one embodiment, from 3 to 30 mol%, from 5 to 20 mol%, or from 8 to 18 mol%. For the phospholipid, a single type may be used alone, or two or more types may be used in combination. When a plurality of types of phospholipids is used, it is preferable that the proportion be the proportion described above as the whole phospholipids. The total lipid is preferably the sum of phospholipids, cationic lipids, amphiphilic compounds, and sterols.

Cationic Lipid

When the payload encapsulated in the lipid nanoparticle is a negatively charged substance (for example, a nucleic acid or the like) and the dispersion medium of the lipid nanoparticle is hydrophilic, a cationic lipid being contained in the lipid nanoparticle enables encapsulation of the payload, and can reduce the particle size of the resulting lipid nanoparticle.

Cationic lipids are roughly composed of a hydrophobic moiety and a hydrophilic moiety. As the constitution thereof, a hydrophobic group such as a fatty acid group or a sterol group is used for the hydrophobic moiety, and a cationic group such as an amino group is used for the hydrophilic moiety. Known examples of the composition of the cationic lipid include a structure in which one hydrophobic group is contained with respect to one hydrophilic group (hereinafter, referred to as "single-chain cationic lipid"), a structure in which two hydrophobic groups are contained with respect to one hydrophilic group (hereinafter, referred to as "double-chain cationic lipid").

The cationic lipid is not particularly limited, and examples thereof include heptadecan-9-yl 8-((2-hydroxyethyl)(6-oxo-6-(undecyloxy)hexyl)amino)octanoate (SM-102), (4-hydroxybutyl)azanediyl)di(hexane-6,1-diyl) bis(2-hexyldecanoate) (ALC-0315), (6Z,9Z,28Z,31Z)-heptatriaconta-6,9,28,31-tetraen-19-yl-4-(dimethylamino)butanoate, (9Z,12Z)-3-((4,4-bis(octyloxy)butanoyl)oxy)-2-((((3-(diethylamino)propoxy)carbonyl)oxy)methyl)propyl octadeca-9,12-dienoate (LP01), di((Z)-non-2-en-1-yl)9-((4-dimethylamino)butanoyl)oxy)heptadecanedioate (L319), 2,2-dilinoleyl-4-dimethylaminoethyl-[1,3]-dioxolane (DLin-KC2-DMA), dilinoleyl-methyl-4-dimethylaminobutyrate (DLin-MC3-DMA), di((Z)-non-2-en-L-yl)9-((4-(dimethylamino)butanoyl)oxy)heptadecanedioate (L319), 1,2-dioleyloxy-N,N-dimethylaminopropane (DODMA), 1,2-dioleoyl-3-dimethylammonium-propane (DODAP), 3β-N-(N',N',dimethyl-aminoethane)-carbamoyl cholesterol (DC-Chol), 1,2-dimyristoyloxypropyl-3-dimethylhydroxyethylammonium (DMRIE), 2,3-dioleyloxy-N-[2(spermine carboxamido)ethyl]-N,N-dimethyl-1-propanaminum trifluoroacetate (DOSPA), 1,2-distearoyl-3-trimethylammoniumpropane (DSTAP), 9,12-octadecadienoic acid, (9Z,12Z)-1,1',1',1'-[(3,6-dioxo-2,5-piperazinediyl) bis(4,1-butanediylnitrilodi-4,1-butanediyl)]ester (OF-C4-Deg-Lin), 1,1'-[[2-[4-[2-[bis(2-hydroxydodecyl)amino]ethyl](2-hydroxydodecyl)amino]ethyl]-1-piperazinyl]ethyl]imino] bis-2-dodecanol (C12-200), 1,2-dilinooleyloxy-N,N-dimethyl-3-aminopropane (DLinDMA), 3,6-bis[4-[bis(2-hydroxydodecyl)amino]butyl]-2,5-piperazinedione (CKK-E12), bis(2-(octyldisulfanyl) ethyl) 3,3'-((4-methyl-11-oxo-12-oxa-15,16-dithia-4,8-diazatetracosyl)azanediyl)dipropionate (306-O12B-3), tetrakis(2-(octyldisulfanyl)ethyl) 3,3',3'',3'''-(((methylazanediyl) bis(propane-3,1-diyl)) bis(azanetolyl))tetrapropionate (306-O12B), 2-(((4-(dimethylamino)butanoyl)oxy)methyl)-2-(((Z)-tetradec-9-enoyl)oxy)methyl)propane-1,3-diyl (9Z,9'Z)-bis(tetradec-9-enoate (TCL053), 3,6-bis[4-[bis[(9Z, 12Z)-2-hydroxy-9,12-octadecadien-1-yl]amino]butyl]-2,5-piperazinedione (OF-02), 1-methyl-4,4-bis[(9Z,12Z)-9,12-octadecadien-1-yloxy]-piperazine (YSK05), N1,N16-didodecyl-4,7,13-tris[3-(dodecylamino)-3-oxopropyl] -4,7,10,13-tetraazahexadecanediamide (98N12-5), alkyl-modified polyamidoamine (PAMAM) dendrimer (G0-C14), N1,N3,N5-tris[3-(didodecylamino)propyl]-1,3,5-benzenetricarbonamide (TT3), 1,2-dimyristoyl-3-trimethylammonium propane chloride (DMTAP), 1-[2-[bis(2-hydroxytridecyl)amino]ethoxy]ethylamino]tridecan-2-ol (C13-112-tri-tail), bis(2-(tetradecylthio)ethyl)3,3'-((3-(1H-imidazole-1-yl)propyl)azanediyl)dipropionate (93-O17S), 3-(didodecylamino)-N1,N1,4-tridodecyl-1-piperazinethanamine (KL10), N1-[2-(didodecylamino) ethyl]-N1,N4,N4-tridodecyl-1,4-piperazinediethanamine (KL22), 14,25-ditridecyl-15,18,21,24-tetraaza-octatriacontane (KL25), 2,2-dilinoleyl-4-dimethylaminomethyl-[1,3]-dioxolane (DLin-K-DMA), 2-({8-[(3β)-cholest-5-en-3-yloxy]octyl} oxy)-N,N-dimethyl-3-[(9Z,12Z)-octadeca-9,12-dien-1-yloxy]propane-1-amine (octyl-CLinDMA), (2R)-2-({8-[(3β)-cholest-5-en-3-yloxy]octyl}oxy)-N,N-dimethyl-3-[(9Z,12Z)octadeca-9,12-dien-1-yloxy]propane-1-amine (octyl-CLinDMA (2R)), stearamidopropyldimethylamine; 1,2-dioleoyl-3-trimethylammonium propane chloride (DOTAP chloride), 1,2-di-O-octadecenyl-3-trimethylammonium propane (chloride salt) (DOTMA), dioctadecyldimethylammonium chloride (DODAC), dioctadecyldimethylammonium chloride (DDAB), and stearyltrimethylammonium chloride. For these cationic lipids, a single type may be used alone or two or more types may be used in combination. Among these, the cationic lipid is preferably a tertiary amine, more preferably a compound having no unsaturated bond in the molecule and having a plurality of ester bonds, still more preferably heptadecan-9-yl 8-((2-hydroxyethyl) (6-oxo-6-(undecyloxy)hexyl)amino) octanoate (SM-102) and/or (4-hydroxybutyl)azanediyl)di(hexane-6,1-diyl) bis(2-hexyldecanoate) (ALC-0315), and yet still more preferably heptadecan-9-yl 8-((2-hydroxyethyl) (6-oxo-6-(undecyloxy)hexyl)amino)octanoate (SM-102).

The cationic lipid is preferably a cationic lipid having two or more hydrophobic moieties having 8 or more carbon atoms (two-chain cationic lipid).

Examples of the cationic lipid include a lipid molecule (ionized lipid) that exhibits charge neutrality at physiological pH (around pH 7.4) and protonates (to $H^+$) in an acidic region (for example, pH 6.5 or less). Such ionizable lipids are usually

tertiary amines. It is a preferred aspect that the cationic lipid is an ionic lipid, from the viewpoint of the ease of functional expression of the anionic payload (in particular, nucleic acid) and the retention in blood. Such a compound may also be used to control the intake into cells.

For these cationic lipids, a single type may be used alone or two or more types may be used in combination.

In one embodiment, the mole ratio of the cationic lipid to the total lipid (preferably the sum of phospholipids, cationic lipids, amphiphilic compounds and sterols) present in the lipid nanoparticle is from 0.5 to 80 mol%, from 0.5 to 75 mol%, from 5 to 75 mol%, from 5 to 70 mol%, or from 10 to 70 mol%. In one embodiment, the mole ratio of the cationic lipid to the total lipid (preferably the sum of phospholipids, cationic lipids, amphiphilic compounds and sterols) present in the lipid nanoparticle may be from 10 to 80 mol%, from 15 to 80 mol%, from 20 to 80 mol%, from 25 to 75 mol%, or from 40 to 75 mol%. When the mole ratio of the cationic lipid is equal to or more than the lower limit, the migration from the endosome into the cytoplasm is likely to proceed, the encapsulation efficiency of the anionic payload (in particular, nucleic acid) improves, and/or the function is likely to be expressed, and when mole ratio is equal to or less than the upper limit, particle formation is likely to be performed.

Sterol

The lipid nanoparticle of the present embodiment preferably contains a sterol.

Examples of the sterol include cholesterol, cholesterol succinic acid, dihydrocholesterol, lanosterol, dihydrolanosterol, desmosterol, stigmasterol, sitosterol, campesterol, brassicasterol, timosterol, ergosterol, campesterol, fucosterol, 22-ketosterol, 20-hydroxycholesterol, 7-hydroxycholesterol, 19-hydroxycholesterol, 22-hydroxycholesterol, 25-hydroxycholesterol, 7-dehydrocholesterol, 5α-cholest-7-en-3β-ol, epicholesterol, dehydroergosterol, sulfate cholesterol, hemisuccinate cholesterol, cholesterol phthalate, cholesterol phosphate, cholesterol valerate, cholesterol hemisuccinate, 3βN-(N',N'-dimethylaminoethane)-carbamoyl cholesterol, cholesterol acetate, cholesteryl olate, cholesteryl linolate, cholesteryl myristate, cholesteryl palmitate, cholesteryl arachidate, cholesterol prostanol, cholesterol esters, cholesteryl phosphorylcholine, and 3,6,9-trioxaoctan-1-ol-cholesteryl-3e-ol. For these sterols, a single type may be used alone, or two or more types may be used in combination. Among these, the sterol is preferably cholesterol.

In one embodiment, the content of sterol with respect to the total lipid (preferably, the total of phospholipids, cationic lipids, amphiphilic compounds, and sterols) is from 5 to 80 mol%, from 10 to 75 mol%, from 15 to 70 mol%, or from 20 to 65 mol%. When the sterol is within the range described above, the lipid nanoparticle is easily formed, and an appropriate particle size is achieved.

In one embodiment, the lipid nanoparticle contains a phospholipid and a sterol, and the content mole ratio of the phospholipid and the sterol is phospholipid : sterol = 1 : 0.1 to 1 : 40, 1 : 1 to 1 : 10, or 1 : 2 to 1 : 5. When the mole ratio is within such a range, lipid nanoparticles having an appropriate particle size are easily formed. In one embodiment, the lipid nanoparticle contains a phospholipid and a sterol, and a content mole ratio of the phospholipid and the sterol is phospholipid : sterol = 1 : 0.05 to 1 : 40, 1 : 0.1 to 1 : 40, 1 : 0.15 to 1 : 15, 1 : 0.15 to 1 : 5, 1 : 0.15 or more and less than 1 : 1, or 1 : 0.5 or more and less than 1 : 1. When the mole ratio of the contents is within such a range, the efficiency of intake into cells improves, and particularly when the payload is a nucleic acid, high expression efficiency is likely to be achieved.

In one embodiment, the modification rate (%) of the lipid nanoparticle with the amphiphilic compound is 0.005% or more and less than 40%, from 0.005 to 30%, from 0.005 to 20%, from 0.05 to 15%, from 0.05 to 10%, or from 0.005 to 5% (from 0.01 to 5%, 0.005% or more and less than 5%, or 0.01% or more and less than 5%), and the content mole ratio of the phospholipid and the sterol is phospholipid : sterol = 1 : 0.1 to 1 : 40, 1 : 0.15 to 1 : 15, 1 : 0.15 to 1 : 5, or 1 : 0.15 or more and less than 1.

In one embodiment, the modification rate (%) of the lipid nanoparticle with the amphiphilic compound is 0.005% or more and less than 40%, from 0.005 to 30%, from 0.005 to 20%, from 0.05 to 15%, from 0.05 to 10%, or from 0.005 to 5% (from 0.01 to 5%, 0.005% or more and less than 5%, or 0.01% or more and less than 5%), the content mole ratio of the phospholipid and the sterol is phospholipid : sterol = 1 : 0.05 to 1 : 40, 1 : 0.1 to 1 : 40, 1 : 0.15 to 1 : 15, 1 : 0.15 to 1 : 5, 1 : 0.15 or more and less than 1 : 1, or 1 : 0.5 or more and less than 1 : 1, and the mole ratio of the cationic lipid to the total lipid (preferably, the sum of phospholipids, cationic lipids, amphiphilic compounds, and sterols) present in the lipid nanoparticle is from 10 to 80 mol%, from 15 to 80 mol%, from 20 to 80 mol%, from 25 to 75 mol%, or from 40 to 75 mol%. In this embodiment, the intake efficiency into cells improves, and high expression efficiency is likely to be achieved particularly when the payload is a nucleic acid.

Payload Encapsulated in Lipid Nanoparticle

The payload encapsulated in the lipid nanoparticle is not particularly limited, but is preferably an anionic compound because it forms a complex with a cationic lipid and is easily introduced into cells. Examples of the anionic compound include nucleic acids; pharmaceuticals such as benzalkonium chloride, betanequol chloride, and pyridostigmine bromide; magnesium ascorbyl phosphate; and sodium ascorbyl phosphate.

The encapsulated amount of the nucleic acid is, for example, from 0.01 to 20 mass%, it may be from 0.05 to 15 mass%, or may be from 0.1 to 10 mass% in the lipid nanoparticle.

In particular, the payload is preferably a nucleic acid because the effect is easily exhibited. That is, one embodiment of the present invention is a lipid nanoparticle containing the amphiphilic compound and encapsulating a nucleic acid. Examples of the nucleic acid include DNA, mRNA, siRNA, microRNA, gapmer, ribozyme, aptamer, artificial nucleic acid, CpG oligodeoxynucleotide, antisense DNA/RNA, and the like.

When the payload is a nucleic acid, in one embodiment, the total number (hereinafter, it is also simply referred to as an N/P ratio) of amine groups (N) of the cationic lipid with respect to the total number of nucleic acid phosphate groups (P) is from 0.01 to 55, from 0.01 to 50, from 0.03 to 50, from 0.05 to 25, from 1 to 25, from 4 to 25, or from 4 to 10. When the payload is a nucleic acid, in one embodiment, the total number (hereinafter, it is also simply referred to as an N/P ratio) of amine groups (N) of the cationic lipid with respect to the total number of nucleic acid phosphate groups (P) is from 0.01 to 55, from 0.03 to 55, from 0.05 to 55, from 1 to 55, from 2 to 55, from 4 to 55, from 10 to 55, or from 20 to 55. Such an N/P ratio is preferable because a complex can be efficiently formed between the cationic lipid nanoparticle and the nucleic acid, and the encapsulation efficiency of the payload can be improved.

When the payload is a nucleic acid, in one embodiment, the mole ratio of the cationic lipid to the nucleic acid is, for example, from 2000 to 200000, and it may be from 5000 to 100000.

Additional Components

The lipid nanoparticle may contain additional components. Examples of the additional components include water, physiological saline, a pharmaceutically acceptable organic solvent, collagen, polyvinyl alcohol, polyvinyl pyrrolidone, a carboxyvinyl polymer, sodium carboxymethyl cellulose salt, sodium polyacrylate, sodium alginate, water-soluble dextran, sodium carboxymethyl starch, pectin, methyl cellulose, ethyl cellulose, xanthan gum, gum arabic, casein, gelatin, agar, diglycerol, propylene glycol, polyethylene glycol, petrolatum, paraffin, stearyl alcohol, stearic acid, human serum albumin, mannitol, sorbitol, lactose, phosphate buffered saline, a biodegradable polymer, a serum-free medium, a surfactant, fat and oil, a pH adjusting agent, and a physiological pH buffer acceptable in vivo. For these additives, a single type may be used alone or two or more types may be used in combination.

Method for Producing Lipid Nanoparticle

The method for producing a lipid nanoparticle is not particularly limited, and a known method can be used. Specifically, when the payload is a nucleic acid, an example thereof is a method in which a phospholipid, a cationic lipid, a sterol, and an amphiphilic compound are dissolved in an organic solvent to produce a lipid solution, and the lipid solution and a nucleic acid solution in which a nucleic acid is dissolved in an aqueous solvent are mixed to produce a dispersion liquid of lipid nanoparticles.

Examples of the aqueous solvent include water and a buffer. Examples of the buffer include phosphate buffered saline (PBS), acetate buffer, citrate buffer, borate buffer, tartrate buffer, Tris buffer, MES buffer, and HEPES buffer. For the aqueous solvent, a single type may be used alone or two or more types may be used in combination. In particular, when the payload is a nucleic acid, the aqueous solvent is preferably a buffer.

The nucleic acid concentration in the aqueous solvent is, for example, from 0.1 to 10000 μg/mL, and it may be from 0.5 to 1000 μg/mL or from 0.5 to 100 μg/mL. The pH of the aqueous solvent is preferably pH 3.5 to 4.5. When the aqueous solvent is added, the temperature of the aqueous solvent is preferably from 20 to 45°C, preferably from 25 to 40°C.

The organic solvent is preferably a polar organic solvent, and examples thereof include ethanol, isopropanol, butanol, and chloroform. For the organic solvent, a single type may be used alone or two or more types may be in combination. Among these, ethanol is preferably used from the viewpoint of particle formability.

The lipid concentration in the organic solvent is, for example, from 0.01 to 100 mg/mL, and it may be from 0.05 to 10 mg/mL, or may be from 0.05 to 1 mg/mL.

Mixing of the lipid solution and the nucleic acid solution can be performed using a microfluidic mixing system (for example, Asia microfluidic system (Syrris) or Nanoassemblr (Precision Nanosystems)). When mixing is performed using a microfluidic mixing system, the flow rate is, for example, from 0.1 to 20 mL/min. The mixing temperature is, for example, from 4 to 45°C, and it may be from 10 to 35°C.

The resulting lipid particles are treated through dialysis or filtration, preferably diafiltration, to remove the organic solvent. After the treatment, the aqueous solution is converted to a buffer at neutral pH, pH 6.8 to pH 7.5, preferably pH 7.2, for example to a PBS buffer. The resulting aqueous solution is preferably sterilized before storage or use, for example by filtration through a 0.22 μm filter.

By mixing the lipid solution and the nucleic acid solution, the hydrophilic portion of the amphiphilic compound and

the phospholipid is disposed on the surface of the particle, the inside of the particle becomes a hydrophobic environment, and the complex of the hydrophobic nucleic acid and the cationic lipid nanoparticle can be encapsulated.

Form

Another aspect of the present invention is a dispersion in which the lipid nanoparticles are dispersed in a dispersion medium. The dispersion is easy to use in a pharmaceutical use suitable as an application. The dispersion medium is preferably an aqueous solvent. The aqueous solvent is not particularly limited, and examples thereof include water (sterile water), physiological saline, phosphate buffered saline (PBS), citrate buffer, Tris buffer, carbonate buffer, and acetate buffer.
The lipid nanoparticles may be in the form of powder or the like produced by freeze-drying or the like after removing the dispersion or the dispersion medium.

Use of Lipid Nanoparticle

The lipid nanoparticle of the present embodiment is excellent in intake into cells, and thus, they can easily deliver a desired component or compound into cells. Thus, the lipid nanoparticle of the present embodiment is preferably a lipid nanoparticle for delivering a payload into cells.
Another embodiment of the present invention is an intracellular delivery composition containing the amphiphilic compound, the nucleic acid, the phospholipid, the sterol, and the cationic lipid. The contents of the respective components in the composition are the same as those described for the lipid nanoparticle.
Further, because the lipid nanoparticle of the present embodiment is excellent in intake into cells, the lipid nanoparticle can be used particularly for pharmaceutical use. That is, another embodiment of the present invention is a pharmaceutical containing the lipid nanoparticle.
The pharmaceutical may be composed of the lipid nanoparticle according to the present disclosure, or may be a composition further containing additional components. Examples of the additional components include water, physiological saline, a pharmaceutically acceptable organic solvent, collagen, polyvinyl alcohol, polyvinyl pyrrolidone, a carboxyvinyl polymer, sodium carboxymethyl cellulose salt, sodium polyacrylate, sodium alginate, water-soluble dextran, sodium carboxymethyl starch, pectin, methyl cellulose, ethyl cellulose, xanthan gum, gum arabic, casein, gelatin, agar, diglycerol, propylene glycol, polyethylene glycol, petrolatum, paraffin, stearyl alcohol, stearic acid, human serum albumin, mannitol, sorbitol, lactose, phosphate buffered saline, a biodegradable polymer, a serum-free medium, a pharmaceutically acceptable surfactant, and a physiological pH buffer acceptable in vivo. For these additives, a single type may be used alone or two or more types may be used in combination.
The pharmaceutical containing the lipid nanoparticle of the present disclosure is preferably used as a liquid preparation or a gel preparation.
The liquid preparation preferably contains water, physiological saline, phosphate buffered saline, citric acid-phosphate buffer, or the like, in addition to the lipid nanoparticle of the present disclosure.
In addition to the lipid nanoparticle of the present disclosure, the gel preparation preferably contains a neutralized anionic polymer such as polyacrylic acid, a thickener such as carboxypolymethylene and carboxymethyl cellulose, pemuren, a polymer emulsifier, and polycarbophil, a lower alcohol such as ethanol or isopropanol, and water.
The lipid nanoparticle or a pharmaceutical (composition) containing the lipid nanoparticle can be utilized for either in vitro or in vivo testing. Further, as a method for administering the lipid nanoparticle or the like to a living body, preferably, parenteral administration, that is, local administration such as intraarticular administration, intravenous administration, intraperitoneal administration, subcutaneous administration, intramuscular administration, intrathecal administration, intracisternal administration, intracerebroventricular administration, or intradermal administration is adopted. It can also be used in ex vivo therapy in which cells and tissues are removed from an organism, treated, and then returned to the original organism. Intravenous or intraperitoneal administration of the (pharmaceutical) composition can also be performed by bolus infusion.
The lipid nanoparticle or the pharmaceutical containing the lipid nanoparticle of the present disclosure is expected to be a drug having high transferability into cells and good retention in blood.

Examples

Next, the present invention will be described in more detail with reference to Examples, but the present invention is not limited to only such Examples.

Measurement of Number-Average Molecular Weight of Polymer

The number-average molecular weight of the polymer produced in Production Examples 1 to 5 described later was measured by gel permeation chromatography (GPC). The measurement conditions in this case were as follows.

Condition for Measuring Number-Average Molecular Weight of Polymer (Polymer Produced in Production Examples 1 to 5)]

- Measuring instrument: manufactured by Tosoh Corporation, product number: HLC-8320GPC
- Molecular weight column: manufactured by Tosoh Corporation, product number: TSKgel SuperAWM-H and Super-AW2500 connected in series (two columns)
- Eluent: 10 mmol/L lithium bromide added dimethylformamide
- Standard substance for calibration curve: polystyrene
- Preparation of measurement solution: A polymer is dissolved in dimethylformamide to prepare a solution having a polymer concentration of 0.2 mass%, and a filtrate prepared by filtering the solution through a filter is used.

Production of Amphiphilic Compound (Polymer)

An amphiphilic compound having a polymer form was produced by the following method.

Production Example 1

A Schlenk flask equipped with a three-way valve was charged with 1.0 g of glycerol monoacrylate, 0.098 g of 1-octadecanethiol, 0.021 g of 2,2'-azobis(isobutyronitrile), 2.25 g of ethanol, and 0.25 g of water. Subsequently, the inside of the flask was replaced with nitrogen, and the mixture was stirred at 80°C for 3 hours. The resulting reaction solution was added dropwise to diethyl ether and purified to produce polyglycerol monoacrylate containing an alkyl group (n-octadecyl group) at its terminal. The resulting amphiphilic compound (polymer) had a number-average molecular weight of 4100. Herein, the molecular weight of the hydrocarbon group having 8 or more carbon atoms (n-octadecyl group) is 253.5.

Production Example 2

A Schlenk flask equipped with a three-way valve was charged with 1.0 g of glycerol monoacrylate, 0.049 g of 1-octadecanethiol, 0.014 g of 2,2'-azobis(isobutyronitrile), 1.8 g of ethanol, and 0.2 g of water. Subsequently, the inside of the flask was replaced with nitrogen, and the mixture was stirred at 80°C for 3 hours. The resulting reaction solution was added dropwise to diethyl ether and purified to produce polyglycerol monoacrylate containing an alkyl group (n-octadecyl group) at its terminal. The resulting amphiphilic compound (polymer) had a number-average molecular weight of 8600. Herein, the molecular weight of the hydrocarbon group having 8 or more carbon atoms (n-octadecyl group) is 253.5.

Production Example 3

A Schlenk flask equipped with a three-way valve was charged with 1.0 g of glycerol monomethacrylate, 0.045 g of 1-octadecanethiol, 0.013 g of 2,2'-azobis(isobutyronitrile), 1.6 g of ethanol, and 0.4 g of n-butanol. Subsequently, the inside of the flask was replaced with nitrogen, and the mixture was stirred at 80°C for 3 hours. The solution portion of the resulting reaction solution was added dropwise to diethyl ether and purified to produce polyglycerol monomethacrylate containing an alkyl group (n-octadecyl group) at its terminal. The resulting amphiphilic compound (polymer) had a number-average molecular weight of 4100. Herein, the molecular weight of the hydrocarbon group having 8 or more carbon atoms (n-octadecyl group) is 253.5.

Production Example 4

A Schlenk flask equipped with a three-way valve was charged with 1.0 g of glycerol monomethacrylate, 0.045 g of 1-octadecanethiol, 0.013 g of 2,2'-azobis(isobutyronitrile), 1.6 g of ethanol, and 0.4 g of n-butanol. Subsequently, the inside of the flask was replaced with nitrogen, and the mixture was stirred at 80°C for 3 hours. The precipitated portion of the resulting reaction solution was dissolved in 1.0 g of ethanol, and further, added dropwise to diethyl ether and purified to produce polyglycerol monomethacrylate containing an alkyl group (n-octadecyl group) at its terminal. The resulting amphiphilic compound (polymer) had a number-average molecular weight of 14000. Herein, the molecular weight of the

hydrocarbon group having 8 or more carbon atoms (n-octadecyl group) is 253.5.

Production Example 5

A Schlenk flask equipped with a three-way valve was charged with 1.0 g of glycerol monomethacrylate, 0.022 g of 4-cyano-4-(phenylcarbonothioylthio)pentanoic acid, 0.016 g of 2,2'-azobis(2,4-dimethylvaleronitrile), 0.8 g of ethanol, and 0.2 g of n-butanol. Subsequently, the inside of the flask was replaced with nitrogen, and the mixture was stirred at 50°C for 30 minutes. The resulting reaction solution was added dropwise to diethyl ether and purified. 0.2 g of the resulting polymer, 0.25 g of propylamine, and 1.0 g of water were placed, stirred overnight at room temperature, and lyophilized to produce polyglycerol monomethacrylate having a thiol group introduced at its terminal. The resulting polymer had a number-average molecular weight of 5200.

Thereafter, 0.0165 g of the polymer produced above, 0.0276 g of distearoyl N-(3-maleimide-1-oxopropyl)-L-$\alpha$-phosphatidylethanolamine (COATSOME (trade name) FE-8080MA3 manufactured by NOF CORPORATION), 75 $\mu$L of triethylamine, 0.06 g of chloroform, and 0.4 g of methanol were placed in a 10 mL screw tube. Subsequently, the mixture was reacted overnight at room temperature, and then added dropwise to diethyl ether and purified to produce polyglycerol monomethacrylate containing lipid at its terminal. The resulting amphiphilic compound (polymer) had a number-average molecular weight of 6000. Herein, the molecular weight of the lipid moiety as a source of the hydrocarbon group having 8 or more carbon atoms is about 954.

Material for Nucleic Acid Encapsulation Test

Nucleic Acid

As the nucleic acid, siRNA and mRNA were used. As the Lusiferase siRNA, a sequence (sense strand: CUUACGCUGAGUACUUCGAdTdT, antisense strand:
UCGAAGUACUCAGCGUAAGdTdT) described in a known document (JP 2010-77091 A) synthesized by Hokkaido System Science Co., Ltd. and annealed was used. Lusiferase mRNA was purchased from TriLink BioTechnologies.

Cationic Lipid

- Stearyltrimethylammonium chloride (FUJIFILM Wako Pure Chemical Corporation)
- SM-102 (Funakoshi Co., Ltd.)

Analysis of Nucleic Acid Encapsulation Efficiency

The nucleic acid encapsulation efficiency and the amount of nucleic acid encapsulation in the lipid particles were determined by the Ribogreen Assay method using Quant-iT (trade name) RiboGreen (trade name) (ThermoFisher SCIENTIFIC). That is, the lipid particle suspension was diluted in each of HEPES buffer A (10 mM HEPES, 300 mM sucrose, pH 7.4) and HEPES buffer B (HEPES buffer A + 0.1% Triton X-100). To these diluted solutions (100 $\mu$L), Quant-iT RiboGreen RNA reagent diluted 2000 fold with TE buffer was added in an equivalent amount, and the fluorescence intensity (excitation wavelength: 485 nm, fluorescence wavelength: 520 nm) was measured using a 96 well plate. Encapsulation efficiency (%) was calculated by the following formula.

$$\text{Encapsulation efficiency (\%)} = (B - A)/B \times 100$$

A (fluorescence intensity in section without Triton X-100), B (fluorescence intensity in section with Triton X-100) The encapsulation amount was calculated from the measured value of each sample obtained by producing a calibration curve from the value obtained by measuring a siRNA or mRNA solution having a known concentration by a Ribogreen Assay method, diluting the siRNA or mRNA solution in HEPES buffer B, and measuring the diluted solution.

Preparation of Lipid Particle

Payload: mRNA

[Example 1] Modification rate of amphiphilic compound 0.1 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 18.2 : 63.6 : 18.1 : 0.1, and the mole ratio of SM-102 to the nucleic acid was 5000 (the N/P ratio of 2.6)) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was centrifuged for 10 minutes at 3000 × G using a centrifugal filter unit (NMWL: 30kDa, Amicon (trade name) Ultra, Merck Corporation) and concentrated, then resuspended in PBS, centrifuged again for 20 minutes at 3000 × G, washed, and stored as a lipid particle suspension at 4°C until use. The particle size of the lipid particles was measured using Zetasizer Ultra (Malvern Paralytical).

[Example 2] Modification rate of amphiphilic compound 0.5 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 18.1 : 63.3 : 18.0 : 0.5, and the mole ratio of SM-102 to the nucleic acid was 5000 (the N/P ratio of 2.6)) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 3] Modification rate of amphiphilic compound 1.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 15.3 : 53.4 : 30.3 : 1.0, and the mole ratio of SM-102 to the nucleic acid was 10000 (the N/P ratio of 5.2)) was dissolved in 99.5 (v/v)% ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 4] Modification rate of amphiphilic compound 5.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 14.7 : 51.2 : 29.1 : 5.0, and the mole ratio of SM-102 to the nucleic acid was 10000 (the N/P ratio of 5.2)) was dissolved in 99.5 (v/v)% ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 5] Modification rate of amphiphilic compound 10.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were

mixed such that the mole ratio was 13.9 : 48.5 : 27.6 : 10.0, and the mole ratio of SM-102 to the nucleic acid was 10000 (the N/P ratio of 5.2)) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 6] Modification rate of amphiphilic compound 20.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 10.6 : 37.1 : 42.2 : 20.0, and the mole ratio of SM-102 to the nucleic acid was 10000 (the N/P ratio of 5.2)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 7] Modification rate of amphiphilic compound 1.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 11.7 : 40.8 : 46.5 : 1.0, and the mole ratio of SM-102 to the nucleic acid was 20000 (the N/P ratio of 10.4)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a nucleic acid solution of about 3.5 μg/mL. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 8] Modification rate of amphiphilic compound 5.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 11.2 : 39.2 : 44.6 : 5.0, and the mole ratio of SM-102 to the nucleic acid was 20000 (the N/P ratio of 10.4)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a nucleic acid solution of about 3.5 μg/mL. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 9] Modification rate of amphiphilic compound 10.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 10.6 : 37.1 : 42.2 : 10.0, and the mole ratio of SM-102 to the nucleic acid was 20000 (the N/P ratio of 10.4)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was

concentrated, washed, and subjected to particle size measurement in the same manner as in Example 1, and then stored at 4°C until use.

[Example 10] Modification rate of amphiphilic compound 20.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 9.4 : 33.0 : 37.6 : 20.0, and the mole ratio of SM-102 to the nucleic acid was 20000 (the N/P ratio of 10.4)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 11] Modification rate of amphiphilic compound 1.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 8.0 : 27.8 : 63.2 : 1.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 12] Modification rate of amphiphilic compound 5.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 7.6 : 26.7 : 60.7 : 5.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 13] Modification rate of amphiphilic compound 10.0 mol% (phospholipid : sterol = about 1 : 3.5 (mole ratio))

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 7.2 : 25.3 : 57.5 : 10.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 14] Modification rate of amphiphilic compound 20.0 mol% (phospholipid : sterol = about 1 : 3.5 (mole ratio))

A lipid mixture (hydrogenated soybean-derived phosphatidylcholine (Avanti Polar Lipids, Inc.), cholesterol

(Tokyo Chemical Industry Co., Ltd.), SM-102 (Funakoshi Co., Ltd.), and the polymer of Production Example 4 were mixed such that the mole ratio was 6.4 : 22.5 : 51.1 : 20.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

Examples 1 to 14 described above are summarized as follows.

[Table 1]

Table 1

| | Phospholipid (mole ratio) | Cholesterol (mole ratio) | SM-102 (mole ratio) | Amphiphilic compound (mole ratio) | Mole ratio of SM-102 to nucleic acid |
|---|---|---|---|---|---|
| Example 1 | 18.2 | 63.6 | 18.1 | 0.1 | 5000 |
| Example 2 | 18.1 | 63.3 | 18 | 0.5 | 5000 |
| Example 3 | 15.3 | 53.4 | 30.3 | 1 | 10000 |
| Example 4 | 14.7 | 51.2 | 29.1 | 5 | 10000 |
| Example 5 | 13.9 | 48.5 | 27.6 | 10 | 10000 |
| Example 6 | 10.6 | 37.1 | 42.2 | 20 | 10000 |
| Example 7 | 11.7 | 40.8 | 46.5 | 1 | 20000 |
| Example 8 | 11.2 | 39.2 | 44.6 | 5 | 20000 |
| Example 9 | 10.6 | 37.1 | 42.2 | 10 | 20000 |
| Example 10 | 9.4 | 33 | 37.6 | 20 | 20000 |
| Example 11 | 8 | 27.8 | 63.2 | 1 | 40000 |
| Example 12 | 7.6 | 26.7 | 60.7 | 5 | 40000 |
| Example 13 | 7.2 | 25.3 | 57.5 | 10 | 40000 |
| Example 14 | 6.4 | 22.5 | 51.1 | 20 | 40000 |

[Comparative Example 1]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 18.2 : 63.6 : 18.1 : 0.1, and the mole ratio of SM-102 to the nucleic acid was 5000) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 2]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name)

DSPE-020CN manufactured by NOF Corporation) were mixed such that the mole ratio was 18.1 : 63.3 : 18.0 : 0.5, and the mole ratio of SM-102 to the nucleic acid was 5000) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 3]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 15.3 : 53.4 : 30.3 : 1.0, and the mole ratio of SM-102 to the nucleic acid was 10000) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 4]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 14.7 : 51.2 : 29.1 : 5.0, and the mole ratio of SM-102 to the nucleic acid was 10000) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 5]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 13.9 : 48.5 : 27.6 : 10.0, and the mole ratio of SM-102 to the nucleic acid was 10000) was dissolved in 99.5% (v/v) ethanol (FUJIFILM Wako Pure Chemical Corporation) to produce a lipid solution of about 0.11 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 6]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 12.4 : 43.1 : 24.5 : 20.0, and the mole ratio of SM-102 to the nucleic acid was 10000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The

resulting dispersion liquid was concentrated, washed, and subjected to particle size measurement in the same manner as in Example 1, and then stored at 4°C until use.

[Comparative Example 7]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 11.7 : 40.8 : 46.5 : 1.0, and the mole ratio of SM-102 to the nucleic acid was 20000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a nucleic acid solution of about 3.5 μg/mL. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 8]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 11.2 : 39.2 : 44.6 : 5.0, and the mole ratio of SM-102 to the nucleic acid was 20000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a nucleic acid solution of about 3.5 μg/mL. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 9]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 10.6 : 37.1 : 42.2 : 10.0, and the mole ratio of SM-102 to the nucleic acid was 20000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 10]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 9.5 : 33.0 : 37.5 : 20.0, and the mole ratio of SM-102 to the nucleic acid was 20000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 11]

A lipid mixture (hydrogenated soybean-derived phosphatidylcholine (Avanti Polar Lipids, Inc.), cholesterol

(Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 8.0 : 27.8 : 63.2 : 1.0, and the mole ratio of SM-102 to the nucleic acid was 40000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 12]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 7.6 : 26.7 : 60.7 : 5.0, and the mole ratio of SM-102 to the nucleic acid was 40000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 13]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 7.2 : 25.3 : 57.5 : 10.0, and the mole ratio of SM-102 to the nucleic acid was 40000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 14]

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and PEG-phospholipid (SUNBRIGHT (trade name) DSPE-020CN manufactured by NOF Corporation) were mixed such that the mole ratio was 6.4 : 22.5 : 51.1 : 20.0, and the mole ratio of SM-102 to the nucleic acid was 40000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 15]

A lipid mixture (hydrogenated soybean-derived phosphatidylcholine (Avanti Polar Lipids, Inc.), cholesterol (Tokyo Chemical Industry Co., Ltd.), and SM-102 (Funakoshi Co., Ltd.) were mixed such that the mole ratio was 11.8 : 41.3 : 46.9, and the mole ratio of SM-102 to the nucleic acid was 20000) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a nucleic acid solution of about 3.5 μg/mL. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid

particles encapsulating a nucleic acid. The resulting dispersion liquid was concentrated, washed, and subjected to particle size measurement in the same manner as in Example 1, and then stored at 4°C until use. The particle size of the particles in the resulting dispersion liquid was the 424 nm.

[Comparative Example 16] Modification rate of amphiphilic compound: 40.0 mol%

A lipid mixture (hydrogenated soybean-derived phosphatidylcholine (Avanti Polar Lipids, Inc.), cholesterol (Tokyo Chemical Industry Co., Ltd.), SM-102 (Funakoshi Co., Ltd.), and the polymer of Production Example 4 were mixed such that the mole ratio was 7.4 : 25.8 : 26.8 : 40.0, and the mole ratio of SM-102 to the nucleic acid was 20000 (the N/P ratio of 10.4)) was dissolved in 99.5% (v/v) ethanol (FUJIFILM Wako Pure Chemical Corporation) to produce a lipid solution of about 0.23 mg/mL. Luciferase mRNA (TriLink BioTechnologies) was dissolved in 25 mM acetate buffer of pH 4.0 to produce a 1.8 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. As a result of concentration, washing, and particle size measurement of the resulting dispersion liquid in the same manner as in Example 1, no particles were formed.

Comparison of Lipid Particle Size

The particle size measurement results of the lipid particles produced in Examples 1 to 14 and Comparative Examples 1 to 14 are shown in Tables 2 and 3. In the lipid particles in which the polymer of Production Example 4 was modified, particles having a size of from 60 to 240 nm were produced regardless of the polymer modification rate and the mole ratio of the cationic lipid (ionized lipid), and in many lipid particles, particles having a size of 150 nm or less were produced. On the other hand, in the lipid particles in which the commercially available PEG lipid was modified, particles having a size of more than 240 nm were produced depending on the polymer modification rate and the mole ratio of the cationic lipid.

[Table 2]

Table 2

| (Unit: nm) | | | | | | | | |
|---|---|---|---|---|---|---|---|---|
| PGLMMA | | N/P ratio | Polymer modification rate (%) | | | | | |
| | | | 0.1 | 0.5 | 1 | 5 | 10 | 20 |
| Cationic lipid/nucleic acid mole ratio | 5000 | 2.6 | 139 | 61 | | | | |
| | 10000 | 5.21 | | | 103 | 104 | 84 | 234 |
| | 20000 | 10.41 | | | 105 | 97 | 122 | 135 |
| | 40000 | 20.82 | | | 162 | 122 | 95 | 174 |

[Table 3]

Table 3

| (Unit: nm) | | | | | | | | |
|---|---|---|---|---|---|---|---|---|
| PEG | | | Polymer modification rate (%) | | | | | |
| | | | 0.1 | 0.5 | 1 | 5 | 10 | 20 |
| Cationic lipid/nucleic acid mole ratio | 5000 | 2.6 | 264 | 215 | | | | |
| | 10000 | 5.21 | | | 250 | 130 | 137 | 99 |
| | 20000 | 10.41 | | | 218 | 203 | 224 | 115 |
| | 40000 | 20.82 | | | 210 | 52 | 203 | 141 |

Storage Stability Test for mRNA-Encapsulating Lipid Particle

For the lipid particles produced in Example 7, Example 8, Example 9, Example 10, and Comparative Example 15 which were suspended in PBS and stored at 4°C, the change in particle size on the third day of storage was measured. For the particle size change, the comparison with the size on the storage start date (Day 0) was performed using the particle size change rate calculated by the following formula.

$$\text{Particle size change rate (\%)} = (B/A) \times 100$$

A (particle size on storage start date (day 0)), B (particle size on the third day of storage)
The results are shown in Table 4.
In the lipid particles of Comparative Example 15 (unmodified), the particle size largely decreased, whereas in the polymer-modified lipid particles of Example 7, Example 8, Example 9, and Example 10, the particle size change was slight.

[Table 4]

Table 4

| (Unit: %) | | | | | |
|---|---|---|---|---|---|
| | Example 7 | Example 8 | Example 9 | Example 10 | Comparative Example 15 |
| Particle size change rate | 105 | 130 | 93 | 134 | 36 |

Cell Introduction Test for mRNA-Encapsulating Lipid Particle Using Cultured Cell

Human breast carcinoma-derived cells (MCF-7, DS Pharma Biomedical Co., Ltd.) were cultured using a DMEM medium (Nacalai Tesque, Inc.) to which fetal bovine serum (FBS) (DS Pharma Biomedical Co., Ltd.) having a final concentration of 10% was added. The cells were seeded on a 100 mm cell culture dish (BD Falcon) at $5.0 \times 10^3$ cell/cm$^2$, and cultured at 37°C, under 5% $CO_2$. MCF-7 cultured in the 100 mm cell culture dish to a 70% confluent state was treated with a 0.25% trypsin/50 mM EDTA solution, and the above-described serum-added DMEM medium was added to stop the trypsin reaction, whereby an MCF-7 cell suspension was produced. The number of cells in the MCF-7 cell suspension was measured using a 0.4 w/v% trypan blue solution (Wako Pure Chemical Industries, Ltd.). The cell suspension was seeded on a 96 well plate (Thermo Fisher Science, Inc.) so that the number of cells per well was $3.2 \times 10^3$, and cultured for 24 hours at 37°C under 5% $CO_2$. After 24 hours, 50 μL of the diluted solution produced with Opti-MEM (Thermo Fisher Science, Inc.) was added to each well so that the nucleic acid encapsulation amount of the lipid particle suspension produced in Example 8, Comparative Example 8, or Comparative Example 15 was 1 μg/mL. Two days after the addition, 50 μL of ONE-Glo (trade name) EX Luciferase Assay System (Promega Corporation) was added per well, and the mixture was stirred at 600 rpm for 3 minutes with a plate shaker, then the amount of luminescence was measured with a plate reader SH-9000 (Corona Electric Co., Ltd.). For the mRNA expression efficiency, the comparison with that of the section with polymer-unmodified lipid particle was performed using the relative amount of luminescence calculated by the following formula.

$$\text{Relative amount of luminescence (\%)} = (B/A) \times 100$$

A (amount of luminescence in the section with polymer-unmodified lipid particles), B (amount of luminescence in the section with the polymer-modified lipid particles)

The results are shown in Fig. 1.

As compared with the lipid particles (unmodified) of Comparative Example 15, the gene expression level decreased in the lipid particles of Comparative Example 8 (modified with PEG phospholipid), whereas the expression level increased in the lipid particles of Example 8.

While the modulator contributes to stabilization of the particles, migration into cells is higher in the unmodified lipid nanoparticles, and thus, the expression efficiency of the payload (for example, nucleic acid) is usually reduced through the modification as in Comparative Example 8. However, unexpectedly, according to the present embodiment, as in Example 8, the expression efficiency of the payload (for example, nucleic acid) was higher than that of the unmodified lipid nanoparticles. This is probably because the invasion (migration) of the lipid nanoparticles of the present embodiment into cells is higher than the invasion (migration) of unmodified or PEG-modified lipid nanoparticles into cells, and the expression efficiency of the payload (for example, nucleic acid) is high after migration into cells.

Payload: siRNA

[Example 15] Modification rate of amphiphilic compound 5.0 mol%

A lipid mixture (hydrogenated soybean-derived phosphatidylcholine (Avanti Polar Lipids, Inc.), cholesterol (Tokyo Chemical Industry Co., Ltd.), stearyltrimethylammonium chloride (FUJIFILM Wako Pure Chemical Corporation), and the polymer of Production Example 4 were mixed such that the mole ratio was 11.0 : 38.4 : 45.7 : 5.0, and the mole ratio of stearyltrimethylammonium chloride to nucleic acid was 250 (the N/P ratio of 0.13)) was dissolved in 99.5% (v/v) ethanol (FUJIFILM Wako Pure Chemical Corporation) to produce a lipid solution of about 0.17 mg/mL. Luciferase siRNA was dissolved in 25 mM acetate buffer of pH 4.0 to produce a 5.7 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 16] Modification rate of amphiphilic compound 10.0 mol%

A lipid mixture (hydrogenated soybean-derived phosphatidylcholine (Avanti Polar Lipids, Inc.), cholesterol (Tokyo Chemical Industry Co., Ltd.), stearyltrimethylammonium chloride (FUJIFILM Wako Pure Chemical Corporation), and the polymer of Production Example 4 were mixed such that the mole ratio was 10.4 : 36.3 : 43.3 : 10.0, and the mole ratio of stearyltrimethylammonium chloride to nucleic acid was 250 (the N/P ratio of 0.13)) was dissolved in 99.5% (v/v) ethanol (FUJIFILM Wako Pure Chemical Corporation) to produce a lipid solution of about 0.17 mg/mL. Luciferase siRNA was dissolved in 25 mM acetate buffer of pH 4.0 to produce a 5.7 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 17] Modification rate of amphiphilic compound 20.0 mol%

A lipid mixture (hydrogenated soybean-derived phosphatidylcholine (Avanti Polar Lipids, Inc.), cholesterol (Tokyo Chemical Industry Co., Ltd.), stearyltrimethylammonium chloride (FUJIFILM Wako Pure Chemical Corporation), and the polymer of Production Example 4 were mixed such that the mole ratio was 9.3 : 32.3 : 38.5 : 20.0, and the mole ratio of stearyltrimethylammonium chloride to nucleic acid was 250 (the N/P ratio of 0.13)) was dissolved in 99.5% ethanol (FUJIFILM Wako Pure Chemical Corporation) to produce a lipid solution of about 0.17 mg/mL. Luciferase siRNA was dissolved in 25 mM acetate buffer of pH 4.0 to produce a 5.7 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Comparative Example 16] Modification rate of amphiphilic compound: 10.0 mol%

A lipid mixture (hydrogenated soybean-derived phosphatidylcholine (Avanti Polar Lipids, Inc.), cholesterol

(Tokyo Chemical Industry Co., Ltd.), stearyltrimethylammonium chloride (FUJIFILM Wako Pure Chemical Corporation), and PEG-phospholipid (SUNBRIGHT (trade name) DSPE-020CN manufactured by NOF Corporation) were mixed such that the mole ratio was 10.4 : 36.3 : 43.3 : 10.0, and the mole ratio of stearyltrimethylammonium chloride to nucleic acid was 250 (the N/P ratio of 0.13)) was dissolved in 99.5% ethanol (FUJIFILM Wako Pure Chemical Corporation) to produce a lipid solution of about 0.17 mg/mL. Luciferase siRNA was dissolved in 25 mM acetate buffer of pH 4.0 to produce a 5.7 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

Cell Introduction Test for siRNA-Encapsulating Lipid Particle Using Cultured Cell

Luciferase-expressing human breast carcinoma-derived cells (MCF-7/Luc, Cosmo Bio Co., Ltd.) were cultured using a DMEM medium (Nacalai Tesque, Inc.) to which fetal bovine serum (FBS) (DS Pharma Biomedical Co., Ltd.) having a final concentration of 10% was added. The cells were seeded on a 100 mm cell culture dish (BD Falcon) at $5.0 \times 10^3$ cell/cm$^2$, and cultured at 37°C, under 5% $CO_2$. MCF-7 cultured in the 100 mm cell culture dish to a 70% confluent state was treated with a 0.25% trypsin/50 mM EDTA solution, and the above-described serum-added DMEM medium was added to stop the trypsin reaction, whereby an MCF-7 cell suspension was produced. The number of cells in the MCF-7 cell suspension was measured using a 0.4 w/v% trypan blue solution (Wako Pure Chemical Industries, Ltd.). The cell suspension was seeded on a 96 well plate (Thermo Fisher Science, Inc.) so that the number of cells per well was $3.2 \times 10^3$, and cultured for 24 hours at 37°C under 5% $CO_2$. After 24 hours, the lipid particle suspension prepared in Example 15, Example 16, Example 17, or Comparative Example 16 was added to each well in a diluted solution prepared with Opti-MEM (Thermo Fisher Science, Inc.) so that the nucleic acid encapsulation amount was 80 pmol/mL, and 50 μL of Opti-MEM alone was added as a control per well. One day after the addition, 50 μL of ONE-Glo (trade name) EX Luciferase Assay System (Promega Corporation) was added per well, and the mixture was stirred at 600 rpm for 3 minutes with a plate shaker, then the amount of luminescence was measured with a plate reader SH-9000 (Corona Electric Co., Ltd.). For the mRNA expression suppression efficiency, the comparison was performed using the relative amount of luminescence calculated by the following formula.

$$\text{Relative amount of luminescence (\%)} = (B/A) \times 100$$

A (amount of luminescence in the section with Opti-MEM alone), B (amount of luminescence in the section with nucleic acid-encapsulating lipid particles)

As a result of comparing the lipid particles produced in Comparative Example 16, Example 15, Example 16, and Example 17 on the gene expression inhibitory efficiency, as shown in Fig. 2, the gene expression inhibitory effect was remarkable by modifying the lipid nanoparticles with the amphiphilic compound of the present embodiment as compared with the lipid nanoparticles modified with PEG phospholipid. Thus, it can be seen that, according to the present embodiment, siRNA is also efficiently taken into cells as in the case of mRNA, and the functional expression is significantly improved.

[Example 18]

A lipid mixture (hydrogenated soybean-derived phosphatidylcholine (Avanti Polar Lipids, Inc.), cholesterol (Tokyo Chemical Industry Co., Ltd.), and the polymer of Production Example 4 were mixed such that the mole ratio was 21.5 : 75.0 : 3.5) was dissolved in 99.5% (v/v) ethanol (FUJIFILM Wako Pure Chemical Corporation) to produce a lipid solution of about 0.17 mg/mL. Luciferase siRNA was dissolved in 25 mM acetate buffer of pH 4.0 to produce a 4.0 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 19]

A lipid mixture (hydrogenated soybean-derived phosphatidylcholine (Avanti Polar Lipids, Inc.), cholesterol (Tokyo Chemical Industry Co., Ltd.), stearyltrimethylammonium chloride (FUJIFILM Wako Pure Chemical Corporation), and the polymer of Production Example 4 were mixed such that the mole ratio was 10.3 : 36.1 : 50.1 : 3.5, and the mole ratio of stearyltrimethylammonium chloride to nucleic acid was 150 (the N/P ratio = 0.08)) was dissolved in 99.5% (v/v) ethanol

(FUJIFILM Wako Pure Chemical Corporation) to produce a lipid solution of about 0.17 mg/mL. Luciferase siRNA was dissolved in 25 mM acetate buffer of pH 4.0 to produce a 4.0 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 20]

A lipid mixture (hydrogenated soybean-derived phosphatidylcholine (Avanti Polar Lipids, Inc.), cholesterol (Tokyo Chemical Industry Co., Ltd.), stearyltrimethylammonium chloride (FUJIFILM Wako Pure Chemical Corporation), and the polymer of Production Example 4 were mixed such that the mole ratio was 10.3 : 36.1 : 50.1 : 3.5, and the mole ratio of stearyltrimethylammonium chloride to nucleic acid was 250 (the N/P ratio = 0.13)) was dissolved in 99.5% (v/v) ethanol (FUJIFILM Wako Pure Chemical Corporation) to produce a lipid solution of about 0.17 mg/mL. Luciferase siRNA was dissolved in 25 mM acetate buffer of pH 4.0 to produce a 4.0 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

Influence of Cationic Lipid on siRNA Encapsulation Efficiency

As a result of comparing the lipid particles produced in Example 18, Example 19, and Example 20 on the siRNA-encapsulation efficiency, the encapsulation efficiency was enhanced by employing a composition in which the mole ratio of the cationic lipid to the nucleic acid is constant (in particular, the N/P ratio is 0.03 or more) as shown in Fig. 3.

[Example 21] Modification rate of amphiphilic compound 0.01 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 28.4 (hydrogenated soybean-derived phosphatidylcholine : dioleoylphosphatidylethanolamine = 1 : 1 in mole ratio) : 19.9 : 51.7 : 0.01, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 22] Modification rate of amphiphilic compound 0.1 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 28.4 (hydrogenated soybean-derived phosphatidylcholine : dioleoylphosphatidylethanolamine = 1 : 1 in mole ratio) : 19.8 : 51.7 : 0.1, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 23] Modification rate of amphiphilic compound 0.45 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry

Co., Ltd.), SM-102 (Funakoshi Co., Ltd.), and the polymer of Production Example 4 were mixed such that the mole ratio was 28.2 (hydrogenated soybean-derived phosphatidylcholine : dioleoylphosphatidylethanolamine = 1 : 1 in mole ratio) : 19.8 : 51.5 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 24] Modification rate of amphiphilic compound 1.5 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 27.9 (hydrogenated soybean-derived phosphatidylcholine : dioleoylphosphatidylethanolamine = 1 : 1 in mole ratio) : 19.6 : 51.0 : 1.5, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 25] Modification rate of amphiphilic compound 5.0 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 27.0 (hydrogenated soybean-derived phosphatidylcholine : dioleoylphosphatidylethanolamine = 1 : 1 in mole ratio) : 18.9 : 49.1 : 5.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 26] Modification rate of amphiphilic compound 10.0 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 25.5 (hydrogenated soybean-derived phosphatidylcholine : dioleoylphosphatidylethanolamine = 1 : 1 in mole ratio) : 17.9 : 46.6 : 10.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 27] Modification rate of amphiphilic compound 20.0 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 22.7 (hydrogenated soybean-derived phosphatidylcholine : dioleoylphosphatidylethanolamine = 1 : 1 in mole ratio) :

15.9 : 41.4 : 20.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 28] Modification rate of amphiphilic compound 30.0 mol% (phospholipid : sterol = about 1 : 0.7 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 19.9 (hydrogenated soybean-derived phosphatidylcholine : dioleoylphosphatidylethanolamine = 1 : 1 in mole ratio) : 13.9 : 36.2 : 30.0, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.27 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 29] Modification rate of amphiphilic compound 0.45 mol% (phospholipid : sterol = about 1 : 0.1 (mole ratio))

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 73.2 (hydrogenated soybean-derived phosphatidylcholine : dioleoylphosphatidylethanolamine = 1 : 1 in mole ratio) : 7.3 : 19.0 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.79 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 30] Phospholipid : sterol = about 1 : 0.2 (mole ratio) (modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 57.9 (hydrogenated soybean-derived phosphatidylcholine : dioleoylphosphatidylethanolamine = 1 : 1 in mole ratio) : 11.6 : 30.1 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.49 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 31] Phospholipid : sterol = about 1 : 0.5 (mole ratio) (modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 35.5 (hydrogenated soybean-derived phosphatidylcholine : dioleoylphosphatidylethanolamine = 1 : 1 in mole ratio) : 17.8 : 46.2 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.30 mg/mL. Luciferase

mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 32] Phospholipid : sterol = about 1 : 1 (mole ratio) (modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 21.6 (hydrogenated soybean-derived phosphatidylcholine : dioleoylphosphatidylethanolamine = 1 : 1 in mole ratio) : 21.6 : 56.3 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.24 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 33] Phospholipid : sterol = about 1 : 4 (mole ratio) (modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 6.5 (hydrogenated soybean-derived phosphatidylcholine : dioleoylphosphatidylethanolamine = 1 : 1 in mole ratio) : 25.8 : 67.3 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.19 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 34] Phospholipid : sterol = about 1 : 10 (mole ratio) (modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 2.7 (hydrogenated soybean-derived phosphatidylcholine : dioleoylphosphatidylethanolamine = 1 : 1 in mole ratio) : 26.9 : 70.0 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.18 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 35] Phospholipid : sterol = about 1 : 20 (mole ratio) (modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 1.4 (hydrogenated soybean-derived phosphatidylcholine : dioleoylphosphatidylethanolamine = 1 : 1 in mole ratio) : 27.3 : 70.9 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.18 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade

name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 36] Phospholipid : sterol = about 1 : 40 (mole ratio) (modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 0.7 (hydrogenated soybean-derived phosphatidylcholine : dioleoylphosphatidylethanolamine = 1 : 1 in mole ratio) : 27.4 : 71.4 : 0.45, and the mole ratio of SM-102 to the nucleic acid was 40000 (the N/P ratio of 20.8)) was dissolved in 99.5% (v/v) ethanol (FUJIFILM Wako Pure Chemical Corporation) to produce a lipid solution of about 0.18 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 37] Cationic lipid mole ratio 12.0 mol% (phospholipid : sterol = about 1 : 0.7 (mole ratio), modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 51.6 (hydrogenated soybean-derived phosphatidylcholine : dioleoylphosphatidylethanolamine = 1 : 1 in mole ratio) : 36.1 : 11.8 : 0.45, and the mole ratio of SM-102 to the nucleic acid was 5000 (the N/P ratio of 2.6)) was dissolved in 99.5% (v/v) ethanol (FUJIFILM Wako Pure Chemical Corporation) to produce a lipid solution of about 0.14 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 38] Cationic lipid mole ratio 21.0 mol% (phospholipid : sterol = about 1 : 0.7 (mole ratio), modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 46.2 (hydrogenated soybean-derived phosphatidylcholine : dioleoylphosphatidylethanolamine = 1 : 1 in mole ratio) : 32.3 : 21.0 : 0.45, and the mole ratio of SM-102 to the nucleic acid was 10000 (the N/P ratio of 5.2)) was dissolved in 99.5% (v/v) ethanol (FUJIFILM Wako Pure Chemical Corporation) to produce a lipid solution of about 0.16 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 39] Cationic lipid mole ratio 35.0 % (phospholipid : sterol = about 1 : 0.7 (mole ratio), modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 38.1 (hydrogenated soybean-derived phosphatidylcholine : dioleoylphosphatidylethanolamine = 1 : 1 in mole ratio) : 26.7 : 34.7 : 0.45, and the mole ratio of SM-102 to the nucleic acid was 20000 (the N/P ratio of 10.4)) was dissolved in 99.5% (v/v) ethanol (FUJIFILM Wako Pure Chemical Corporation) to produce a lipid solution of about 0.19 mg/mL. Luciferase mRNA (TriLink BioTechnologies) was dissolved in 25 mM acetate buffer of pH 4.0 to produce a 3.5 μg/mL nucleic acid

solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 40] Cationic lipid mole ratio 61.0 % (phospholipid : sterol = about 1 : 0.7 (mole ratio), modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 22.5 (hydrogenated soybean-derived phosphatidylcholine : dioleoylphosphatidylethanolamine = 1 : 1 in mole ratio) : 15.7 : 61.4 : 0.45, and the mole ratio of SM-102 to the nucleic acid was 60000 (the N/P ratio of 31.2)) was dissolved in 99.5% (v/v) ethanol (FUJIFILM Wako Pure Chemical Corporation) to produce a lipid solution of about 0.34 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 41] Cationic lipid mole ratio 68.0 % (phospholipid : sterol = about 1 : 0.7 (mole ratio), modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 18.6 (hydrogenated soybean-derived phosphatidylcholine : dioleoylphosphatidylethanolamine = 1 : 1 in mole ratio) : 13.0 : 67.9 : 0.45, and the mole ratio of SM-102 to the nucleic acid was 80000 (the N/P ratio of 41.6)) was dissolved in 99.5% (v/v) ethanol (FUJIFILM Wako Pure Chemical Corporation) to produce a lipid solution of about 0.41 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

[Example 42] Cationic lipid mole ratio 72.0 % (phospholipid : sterol = about 1 : 0.7 (mole ratio), modification rate of amphiphilic compound: 0.45 mol%)

A lipid mixture (phospholipid (hydrogenated soybean-derived phosphatidylcholine (Avanti Polar Lipids, Inc.), dioleoylphosphatidylethanolamine (FUJIFILM Wako Pure Chemical Corporation)), cholesterol (Tokyo Chemical Industry Co., Ltd.), SM-102 (Funakoshi Co., Ltd.), and the polymer of Production Example 4 were mixed such that the mole ratio was 15.9 (hydrogenated soybean-derived phosphatidylcholine : dioleoylphosphatidylethanolamine = 1 : 1 in mole ratio) : 11.1 : 72.5 : 0.45, and the mole ratio of SM-102 to the nucleic acid was 100000 (the N/P ratio of 52.1)) was dissolved in 99.5% (v/v) ethanol (FUJIFILM Wako Pure Chemical Corporation) to produce a lipid solution of about 0.49 mg/mL. Luciferase mRNA (TriLink BioTechnologies) was dissolved in 25 mM acetate buffer of pH 4.0 to produce a 3.5 μg/mL nucleic acid solution. The resulting lipid solution and nucleic acid solution were mixed at room temperature by NanoAssemblr (trade name) (Precision NanoSystems) at a flow rate ratio of 1.33 mL/min : 1.33 mL/min to produce a dispersion liquid containing lipid particles encapsulating a nucleic acid. The resulting dispersion liquid was concentrated, washed, and subjected to particle size measurement in the same manner as in Example 1, and then stored at 4°C until use.

Examples 21 to 42 described above are summarized as follows.

[Table 5]

[Table 5]

| | | | Phospholipid (mole ratio) | Sterol (mole ratio) | SM-102 (mole ratio) | Amphiphilic compound (mole ratio) | Mole ratio of SM-102 to nucleic acid |
|---|---|---|---|---|---|---|---|
| Example 21 | Amphiphilic compound (mole ratio) | 0.01 | 28.4 | 19.9 | 51.7 | 0.01 | 40000 |
| Example 22 | | 0.1 | 28.4 | 19.8 | 51.7 | 0.1 | 40000 |
| Example 23 | | 0.45 | 28.2 | 19.8 | 51.5 | 0.45 | 40000 |
| Example 24 | | 1.5 | 27.9 | 19.6 | 51 | 1.5 | 40000 |
| Example 25 | | 5 | 27 | 18.9 | 49.1 | 5 | 40000 |
| Example 26 | | 10 | 25.5 | 17.9 | 46.6 | 10 | 40000 |
| Example 27 | | 20 | 22.7 | 15.9 | 41.4 | 20 | 40000 |
| Example 28 | | 30 | 19.9 | 13.9 | 36.2 | 30 | 40000 |
| Example 29 | Phospholipid sterol ratio | 0.1 | 73.2 | 7.3 | 19.0 | 0.45 | 40000 |
| Example 30 | | 0.2 | 57.9 | 11.6 | 30.1 | 0.45 | 40000 |
| Example 31 | | 0.5 | 35.5 | 17.8 | 46.2 | 0.45 | 40000 |
| Example 24 | | 0.7 | 27.9 | 19.6 | 51 | 1.5 | 40000 |
| Example 32 | | 1 | 21.6 | 21.6 | 56.3 | 0.45 | 40000 |
| Example 33 | | 4 | 6.5 | 25.8 | 67.3 | 0.45 | 40000 |
| Example 34 | | 10 | 2.7 | 26.9 | 70.0 | 0.45 | 40000 |
| Example 35 | | 20 | 1.4 | 27.3 | 70.9 | 0.45 | 40000 |
| Example 36 | | 40 | 0.7 | 27.4 | 71.4 | 0.45 | 40000 |
| Example 37 | Cationic lipid mole ratio | 12 | 51.6 | 36.1 | 11.8 | 0.45 | 5000 |
| Example 38 | | 21 | 46.2 | 32.3 | 21.0 | 0.45 | 10000 |
| Example 39 | | 35 | 38.1 | 26.7 | 34.7 | 0.45 | 20000 |
| Example 24 | | 51 | 27.9 | 19.6 | 51 | 1.5 | 40000 |
| Example 40 | | 61 | 22.5 | 15.7 | 61.4 | 0.45 | 60000 |
| Example 41 | | 68 | 18.6 | 13.0 | 67.9 | 0.45 | 80000 |
| Example 42 | | 72 | 15.9 | 11.1 | 72.5 | 0.45 | 100000 |

Nucleic Acid Recovery Rate Comparative Test

The nucleic acid recovery rate was calculated with the following formula.

Nucleic acid recovery rate [%] = (amount of nucleic acid recovered by destroying produced lipid nanoparticles ($\mu$g)/total amount of nucleic acid used for production of lipid nanoparticles ($\mu$g)) $\times$ 100

Here, the "amount of nucleic acid recovered by destroying the produced lipid nanoparticles" was calculated from the product of the nucleic acid concentration and the recovered lipid nanoparticle suspension amount. Here, the nucleic acid concentration was calculated by the same method as the encapsulation amount described in the section of the encapsulation efficiency.

The nucleic acid recovery rates of Comparative Example 15 and Example 21 to Example 42 are shown in Fig. 4. The nucleic acid recovery rate in each of the LNPs produced in Examples 21 to 42 was higher than that in Comparative Example 15. Focusing on the polymer modification rate, while extremely high recovery rates were shown in Examples 23 to 28 where the modification rate was high, surprisingly, a remarkable improvement in recovery rates was also observed in Examples 21 and 22, where the modification rate was low, as compared with Comparative Example 15. Focusing on the phospholipid sterol ratio, in all the examined ranges of Example 29 to Example 36, a remarkable improvement in recovery rate was observed as compared with Comparative Example 15.

Gene Expression Test Using Lipid Nanoparticles with Different Polymer Modification Rates

Human breast carcinoma-derived cells (MCF-7, DS Pharma Biomedical Co., Ltd.) were cultured using a DMEM medium (Nacalai Tesque, Inc.) to which fetal bovine serum (FBS) (DS Pharma Biomedical Co., Ltd.) having a final concentration of 10% was added. The cells were seeded on a 100 mm cell culture dish (BD Falcon) at $5.0 \times 10^3$ cell/cm$^2$, and cultured at 37°C, under 5% $CO_2$. MCF-7 cultured in the 100 mm cell culture dish to a 70% confluent state was treated with a 0.25% trypsin/50 mM EDTA solution, and the above-described serum-added DMEM medium was added to stop the trypsin reaction, whereby an MCF-7 cell suspension was produced. The number of cells in the MCF-7 cell suspension was measured using a 0.4 w/v% trypan blue solution (Wako Pure Chemical Industries, Ltd.). The cell suspension was seeded on a 96 well plate (Thermo Fisher Science, Inc.) so that the number of cells per well was $3.2 \times 10^3$, and cultured for 24 hours at 37°C under 5% $CO_2$. After 24 hours, the medium was changed from the DMEM medium to a serum-free DMEM medium from which FBS was removed, and to each well, 50 µL of a diluted solution prepared by using the lipid particle suspension prepared in the corresponding one of Examples 21 to 28 and Comparative Example 15 in the serum-free DMEM medium so that the nucleic acid encapsulation amount was 200 ng/mL was added. Two days after the addition, 50 µL of ONE-Glo (trade name) EX Luciferase Assay System (Promega Corporation) was added per well, and the mixture was stirred at 600 rpm for 3 minutes with a plate shaker, then the amount of luminescence was measured with a plate reader SH-9000 (Corona Electric Co., Ltd.). For the expression efficiency of mRNA, the test sections were compared by using the relative amount of luminescence calculated by the following formula.

$$\text{Relative amount of luminescence (\%)} = (B/A) \times 100$$

A (amount of luminescence in the section with the lipid particles of Comparative Example 15), B (amount of luminescence in the section with the lipid particles of each polymer modification rate)

The results are shown in Fig. 5.

In the lipid particles of Examples 21 to 28, the gene expression level was equivalent or increased as compared with the lipid particles of Comparative Example 15. In particular, high gene expression levels were shown in Examples 21 to 25 in which the polymer modification rate was low, and the amount of luminescence of the lipid particles produced in Example 21 was about 170 times the amount of luminescence of the lipid particles produced in Example 8. In general, it is known that when the polymer modification rate to lipid particles decreases, the particles aggregate and the particle diameter increases. However, unexpectedly, in the lipid particles of Example 21, the particle size maintained a good size of 200 nm or less despite the extremely low modification rate of 0.01%. Conversely, when the polymer modification rate is high, the stability of the particles is deteriorated, and it is difficult to maintain the particle shape, but lipid particles maintaining an appropriate particle size were confirmed even when the polymer modification rate was 30%.

Gene Expression Test with Lipid Nanoparticles under Conditions of Different pH Values

A powdered DMEM medium (D2902, Merck Corporation) was dissolved in 1 L of ultrapure water sterilized with high-pressure steam, and sodium hydrogen carbonate (Wako Pure Chemical Industries, Ltd.) was added thereto to adjust the pH to 6.7, and then the solution was sterilized by filtration to produce a low-pH-DMEM medium (pH 6.7).

Human breast carcinoma-derived cells (MCF-7, DS Pharma Biomedical Co., Ltd.) were cultured using a DMEM medium (Nacalai Tesque, Inc.) to which fetal bovine serum (FBS) (DS Pharma Biomedical Co., Ltd.) having a final concentration of 10% was added. The cells were seeded on a 100 mm cell culture dish (BD Falcon) at $5.0 \times 10^3$ cell/cm$^2$, and cultured at 37°C, under 5% $CO_2$. MCF-7 cultured in the 100 mm cell culture dish to a 70% confluent state was treated with a 0.25% trypsin/50 mM EDTA solution, and the above-described serum-added DMEM medium was added to stop the trypsin reaction, whereby an MCF-7 cell suspension was produced. The number of cells in the MCF-7 cell suspension was measured using a 0.4 w/v% trypan blue solution (Wako Pure Chemical Industries, Ltd.). The cell suspension was seeded on a 96 well plate (Thermo Fisher Science, Inc.) so that the number of cells per well was $3.2 \times 10^3$, and cultured for 24 hours at 37°C under 5% $CO_2$. After 24 hours, the DMEM medium was removed from the well plate, the medium was replaced with a low-pH-DMEM medium, and to each well, 50 µL of a diluted solution prepared by using the lipid particle suspension prepared in the corresponding one of Examples 21 to 28 and Comparative Example 8 in the serum-free DMEM medium so that the nucleic acid encapsulation amount was 200 ng/mL was added. Two days after the addition, 50 µL of ONE-Glo (trade name) EX Luciferase Assay System (Promega Corporation) was added per well, and the mixture was stirred at 600 rpm for 3 minutes with a plate shaker, then the amount of luminescence was measured with a plate reader SH-9000 (Corona Electric Co., Ltd.). For the expression efficiency of mRNA, the test sections were compared by using the relative amount of luminescence calculated by the following formula.

$$\text{Relative amount of luminescence (\%)} = (B/A) \times 100$$

A (amount of luminescence in the section with the lipid particles of Comparative Example 15), B (amount of luminescence in the section with the lipid particles of each polymer modification rate)

The results are shown in Fig. 6.

Even in a situation where the pH of the medium was low, comparable or more remarkable gene expression levels were confirmed in the lipid particles of Examples 21 to 28 as compared with the lipid particles of Comparative Example 15. In general, it is known that the extracellular pH around tumor tissue is usually lower (pH 6.2 to 6.8) than the extracellular pH (7.4) of normal tissue, and it is effective from the viewpoint of treatment that gene expression ability can be maintained even in a low pH environment.

Gene Expression Test Using Lipid Nanoparticles with Different Phospholipid Sterol Ratios

Human breast carcinoma-derived cells (MCF-7, DS Pharma Biomedical Co., Ltd.) were cultured using a DMEM medium (Nacalai Tesque, Inc.) to which fetal bovine serum (FBS) (DS Pharma Biomedical Co., Ltd.) having a final concentration of 10% was added. The cells were seeded on a 100 mm cell culture dish (BD Falcon) at $5.0 \times 10^3$ cell/cm$^2$, and cultured at 37°C, under 5% $CO_2$. MCF-7 cultured in the 100 mm cell culture dish to a 70% confluent state was treated with a 0.25% trypsin/50 mM EDTA solution, and the above-described serum-added DMEM medium was added to stop the trypsin reaction, whereby an MCF-7 cell suspension was produced. The number of cells in the MCF-7 cell suspension was measured using a 0.4 w/v% trypan blue solution (Wako Pure Chemical Industries, Ltd.). The cell suspension was seeded on a 96 well plate (Thermo Fisher Science, Inc.) so that the number of cells per well was $3.2 \times 10^3$, and cultured for 24 hours at 37°C under 5% $CO_2$. After 24 hours, the medium was changed from the DMEM medium to a serum-free DMEM medium from which FBS was removed, and to each well, 50 μL of a diluted solution prepared by using the lipid particle suspension prepared in the corresponding one of Example 24, Examples 29 to 36, and Comparative Example 15 in the serum-free DMEM medium so that the nucleic acid encapsulation amount was 200 ng/mL was added. Two days after the addition, 50 μL of ONE-Glo (trade name) EX Luciferase Assay System (Promega Corporation) was added per well, and the mixture was stirred at 600 rpm for 3 minutes with a plate shaker, then the amount of luminescence was measured with a plate reader SH-9000 (Corona Electric Co., Ltd.). For the expression efficiency of mRNA, the test sections were compared by using the relative amount of luminescence calculated by the following formula.

$$\text{Relative amount of luminescence (\%)} = (B/A) \times 100$$

A (amount of luminescence in the section with the lipid particles of Comparative Example 15), B (amount of luminescence in the section with the lipid particles having each phospholipid sterol ratio)

The results are shown in Fig. 7.

In the lipid particles of Example 24 and Examples 29 to 36, the gene expression level was significantly increased as compared with the lipid particles of Comparative Example 15. In addition, all the lipid particles had a particle size in the range of from 30 to 200 nm, and satisfactory particles were generated.

Gene Expression Test Using Lipid Nanoparticles Having Different Cationic Ratios

Human breast carcinoma-derived cells (MCF-7, DS Pharma Biomedical Co., Ltd.) were cultured using a DMEM medium (Nacalai Tesque, Inc.) to which fetal bovine serum (FBS) (DS Pharma Biomedical Co., Ltd.) having a final concentration of 10% was added. The cells were seeded on a 100 mm cell culture dish (BD Falcon) at $5.0 \times 10^3$ cell/cm$^2$, and cultured at 37°C, under 5% $CO_2$. MCF-7 cultured in the 100 mm cell culture dish to a 70% confluent state was treated with a 0.25% trypsin/50 mM EDTA solution, and the above-described serum-added DMEM medium was added to stop the trypsin reaction, whereby an MCF-7 cell suspension was produced. The number of cells in the MCF-7 cell suspension was measured using a 0.4 w/v% trypan blue solution (Wako Pure Chemical Industries, Ltd.). The cell suspension was seeded on a 96 well plate (Thermo Fisher Science, Inc.) so that the number of cells per well was $3.2 \times 10^3$, and cultured for 24 hours at 37°C under 5% $CO_2$. After 24 hours, the medium was changed from the DMEM medium to a serum-free DMEM medium from which FBS was removed, and to each well, 50 μL of a diluted solution prepared by using the lipid particle suspension prepared in the corresponding one of Example 24, Examples 37 to 42, and Comparative Example 15 in the serum-free DMEM medium so that the nucleic acid encapsulation amount was 200 ng/mL was added. Two days after the addition, 50 μL of ONE-Glo (trade name) EX Luciferase Assay System (Promega Corporation) was added per well, and the mixture was stirred at 600 rpm for 3 minutes with a plate shaker, then the amount of luminescence was measured with a plate reader SH-9000 (Corona Electric Co., Ltd.). For the expression efficiency of mRNA, the test sections were compared by using the relative amount of luminescence calculated by the following formula.

$$\text{Relative amount of luminescence (\%)} = (B/A) \times 100$$

A (amount of luminescence in the section with the lipid particles of Comparative Example 15), B (amount of luminescence in the section with the lipid particles of each cationic ratio)

The results are shown in Fig. 8.

In the lipid particles of Example 24 and Examples 37 to 42, the gene expression level was significantly increased as compared with the lipid particles of Comparative Example 15. In addition, all the lipid particles had a particle size in the range of from 30 to 200 nm, and satisfactory particles were generated.

Gene Expression Test in Different Cell Types

Human lung cancer-derived cells (A549, DS Pharma Biomedical Co., Ltd.) and human pancreatic cancer-derived cells (S2-013, obtained from Cell Resource Center for Biomedical Research, Institute of Development, Aging and Cancer, Tohoku University) were used to perform a gene expression test. Culture was performed in the same manner as in the test using MCF-7 except for the difference in cell type. For each cell seeded in a 96 well plate (Thermo Fisher Science, Inc.), to each well, 50 $\mu$L of a diluted solution prepared by using the lipid particle suspension prepared in Example 24 or Comparative Example 15 in a serum-free DMEM medium so that the nucleic acid encapsulation amount was 200 ng/mL was added. Two days after the addition, 50 $\mu$L of ONE-Glo (trade name) EX Luciferase Assay System (Promega Corporation) was added per well, and the mixture was stirred at 600 rpm for 3 minutes with a plate shaker, then the amount of luminescence was measured with a plate reader SH-9000 (Corona Electric Co., Ltd.). For the expression efficiency of mRNA, the test sections were compared by using the relative amount of luminescence calculated by the following formula.

$$\text{Relative amount of luminescence (\%)} = (B/A) \times 100$$

A (amount of luminescence in the section with the lipid particles of Comparative Example 15), B (amount of luminescence in the section with the lipid particles of each cell)

The results are shown in Fig. 9.

In all the cells used, the gene expression level was significantly increased in the lipid particles of Example 24 as compared with the lipid particles of Comparative Example 15.

The present application is based on Japanese Patent Application No. 2023-121431 filed on July 26, 2023, the disclosure content of which is incorporated herein by reference in its entirety.

**Claims**

1. A lipid nanoparticle comprising an amphiphilic compound, wherein the amphiphilic compound includes:

   (i) a site (I) containing a constituent unit (A) derived from a monomer (a) having two or more hydroxy groups in a molecule and having from 2 to 10 carbon atoms constituting a side chain among carbon atoms of the constituent unit; and
   (ii) a hydrocarbon group having 8 or more carbon atoms.

2. The lipid nanoparticle according to claim 1, wherein a nucleic acid is encapsulated in the lipid nanoparticle.

3. The lipid nanoparticle according to claim 1 or 2,
   wherein the constituent unit (A) is a constituent unit represented by Chemical Formula (1):

[Chem. 1]

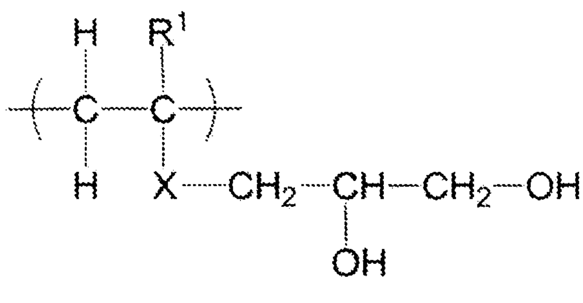

(1)

where

$R^1$ represents a hydrogen atom or a methyl group,
X represents -C(=O)-O-, -C(=O)-NH-, -O-, $-CH_2O-$, or $-CH_2CH_2O-$.

**4.** The lipid nanoparticle according to claim 3, wherein the constituent unit (A) has a proportion in the site (I) of from 80 to 100 mass%.

**5.** The lipid nanoparticle according to claim 1 or 2, wherein the amphiphilic compound has a modification rate (%) of 0.01% or more and less than 40%, the modification rate being calculated by a formula shown below.

Modification rate (%) = {content (mol) of the amphiphilic compound/total amount (mol) of lipids contained in the lipid nanoparticle} × 100 (%)

**6.** The lipid nanoparticle according to claim 1 or 2, further comprising a cationic lipid.

**7.** The lipid nanoparticle according to claim 6, wherein the cationic lipid has a mole ratio to a total lipid present in the lipid nanoparticle of from 0.5 to 75 mol%.

**8.** The lipid nanoparticle according to claim 1 or 2, further comprising a phospholipid.

**9.** The lipid nanoparticle according to claim 8, further comprising a sterol,
wherein the phospholipid and the sterol have a content mole ratio of phospholipid : sterol = 1 : 0.1 to 1 : 40.

**10.** The lipid nanoparticle according to claim 1 or 2, wherein a particle size of the lipid nanoparticle is from 10 to 300 nm.

**11.** A pharmaceutical comprising the lipid nanoparticle described in claim 1 or 2.

**12.** A method for producing a lipid nanoparticle,

the lipid nanoparticle containing a phospholipid, a sterol, a cationic lipid, and an amphiphilic compound,
the amphiphilic compound including (i) a site (I) containing a constituent unit (A) derived from a monomer (a) having two or more hydroxy groups in a molecule and having from 2 to 10 carbon atoms constituting a side chain among carbon atoms of the constituent unit; and (ii) a hydrocarbon group having 8 or more carbon atoms,
the method comprising:

mixing the phospholipid, the sterol, the cationic lipid, and the amphiphilic compound with a solvent; and
bringing the resulting mixture into contact with a payload to produce a lipid nanoparticle encapsulating the payload.

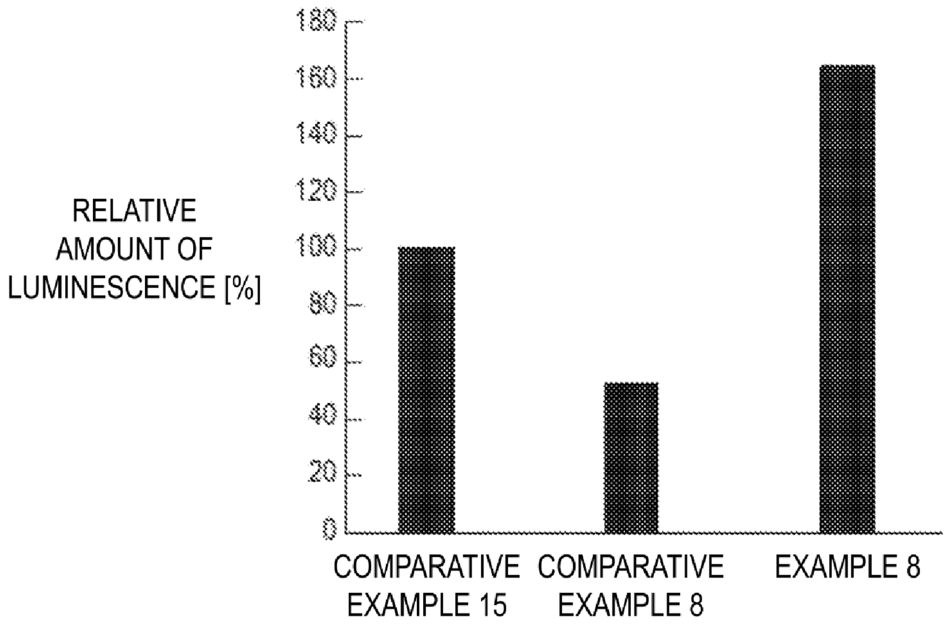
180
160
140
120
100
80
60
40
20
0
RELATIVE AMOUNT OF LUMINESCENCE [%]
COMPARATIVE EXAMPLE 15
COMPARATIVE EXAMPLE 8
EXAMPLE 8

FIG. 1

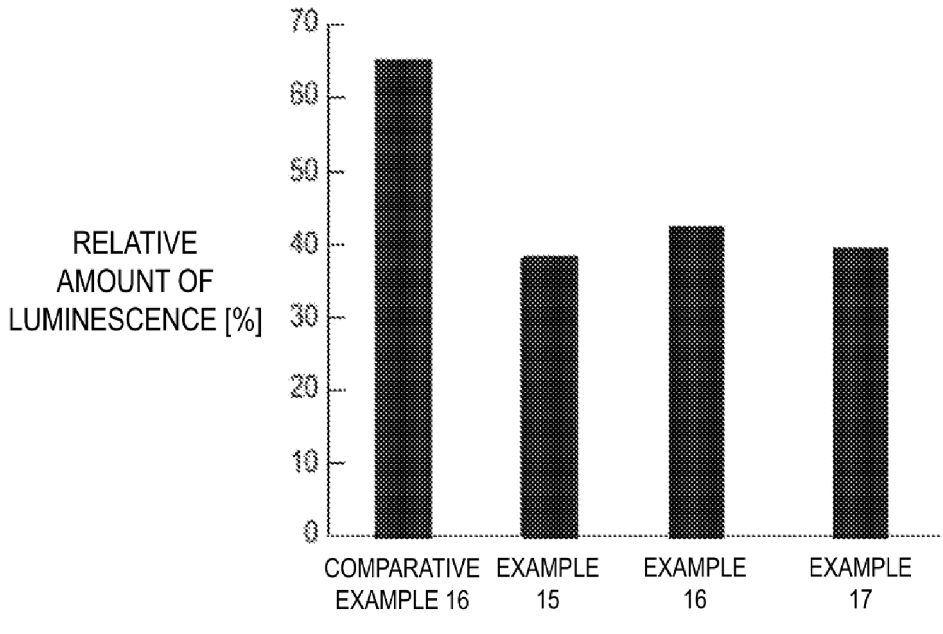
70
60
50
40
30
20
10
0
RELATIVE AMOUNT OF LUMINESCENCE [%]
COMPARATIVE EXAMPLE 16
EXAMPLE 15
EXAMPLE 16
EXAMPLE 17

FIG. 2

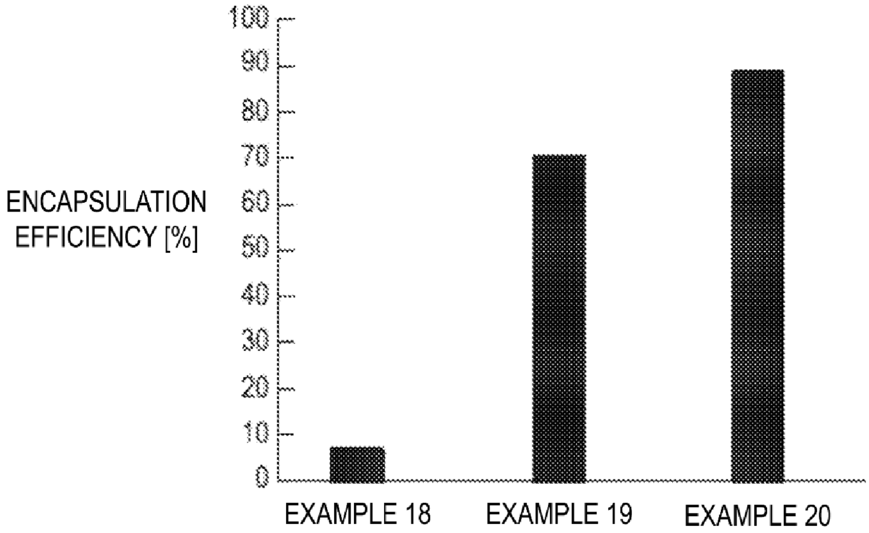
ENCAPSULATION EFFICIENCY [%]
100
90
80
70
60
50
40
30
20
10
0
EXAMPLE 18
EXAMPLE 19
EXAMPLE 20

FIG. 3

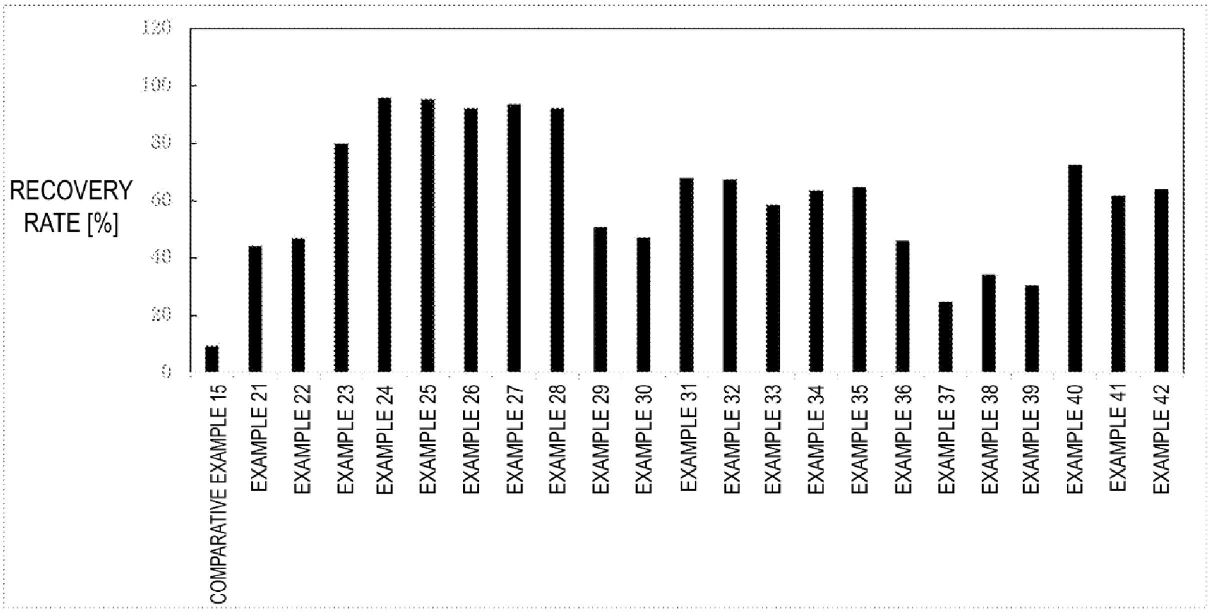
RECOVERY RATE [%]
120
100
80
60
40
20
0
COMPARATIVE EXAMPLE 15
EXAMPLE 21
EXAMPLE 22
EXAMPLE 23
EXAMPLE 24
EXAMPLE 25
EXAMPLE 26
EXAMPLE 27
EXAMPLE 28
EXAMPLE 29
EXAMPLE 30
EXAMPLE 31
EXAMPLE 32
EXAMPLE 33
EXAMPLE 34
EXAMPLE 35
EXAMPLE 36
EXAMPLE 37
EXAMPLE 38
EXAMPLE 39
EXAMPLE 40
EXAMPLE 41
EXAMPLE 42

FIG. 4

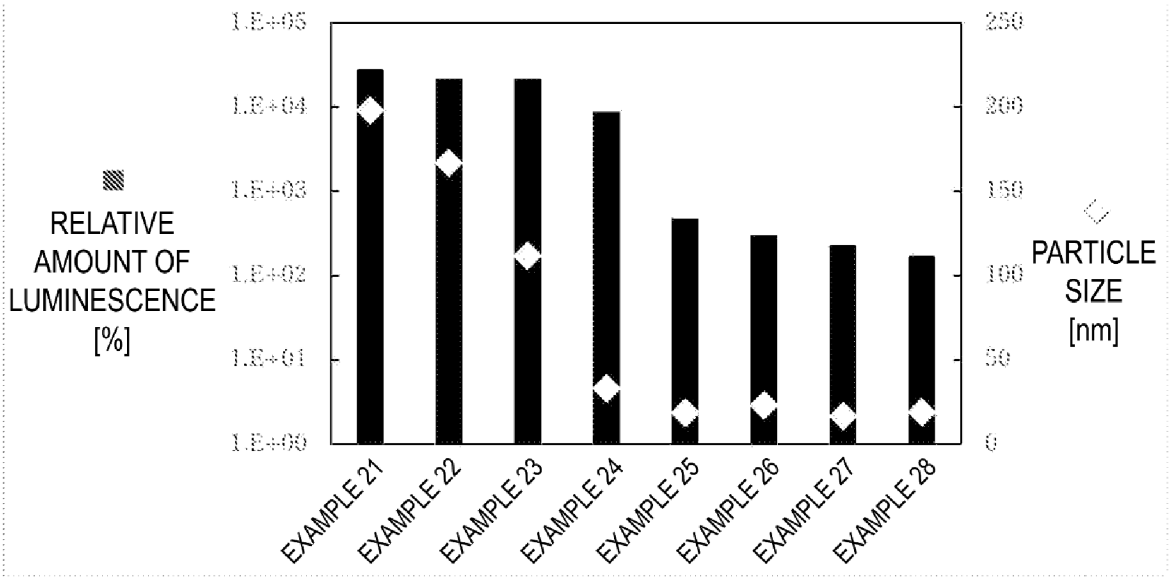
1.E+05
1.E+04
1.E+03
1.E+02
1.E+01
1.E+00
RELATIVE AMOUNT OF LUMINESCENCE [%]
250
200
150
100
50
0
PARTICLE SIZE [nm]
EXAMPLE 21
EXAMPLE 22
EXAMPLE 23
EXAMPLE 24
EXAMPLE 25
EXAMPLE 26
EXAMPLE 27
EXAMPLE 28

FIG. 5

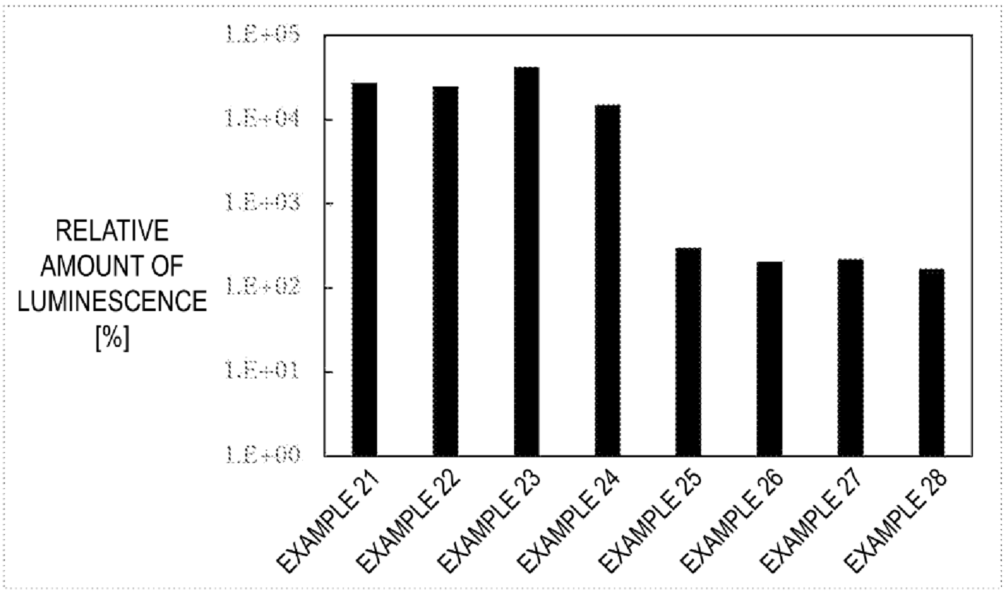
1.E+05
1.E+04
1.E+03
1.E+02
1.E+01
1.E+00
RELATIVE AMOUNT OF LUMINESCENCE [%]
EXAMPLE 21
EXAMPLE 22
EXAMPLE 23
EXAMPLE 24
EXAMPLE 25
EXAMPLE 26
EXAMPLE 27
EXAMPLE 28

FIG. 6

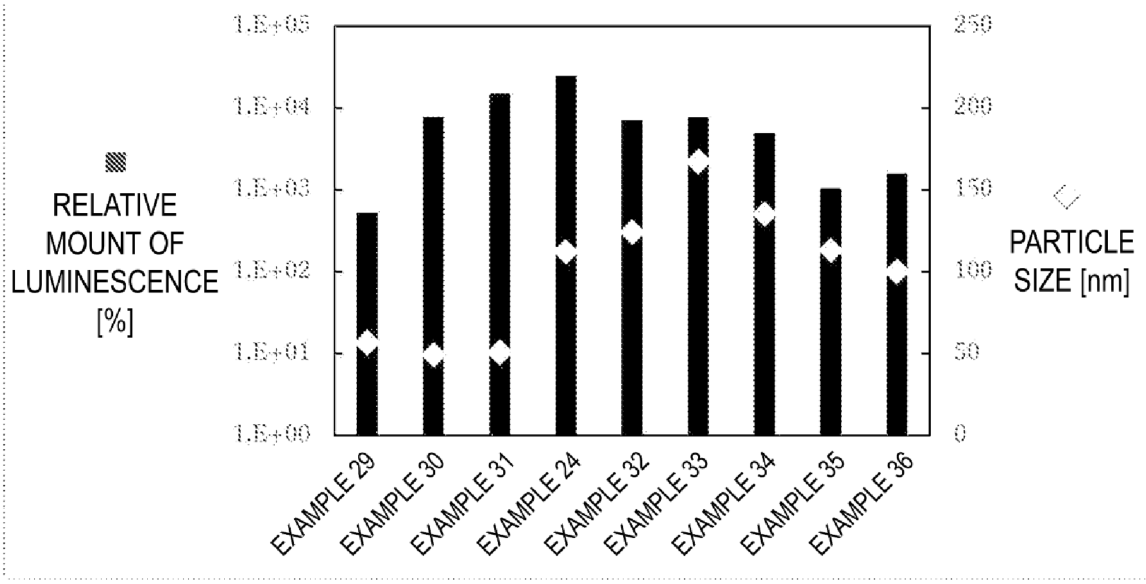
RELATIVE
MOUNT OF
LUMINESCENCE
[%]
1.E+05
1.E+04
1.E+03
1.E+02
1.E+01
1.E+00
250
200
150
100
50
0
PARTICLE
SIZE [nm]
EXAMPLE 29
EXAMPLE 30
EXAMPLE 31
EXAMPLE 24
EXAMPLE 32
EXAMPLE 33
EXAMPLE 34
EXAMPLE 35
EXAMPLE 36

FIG. 7

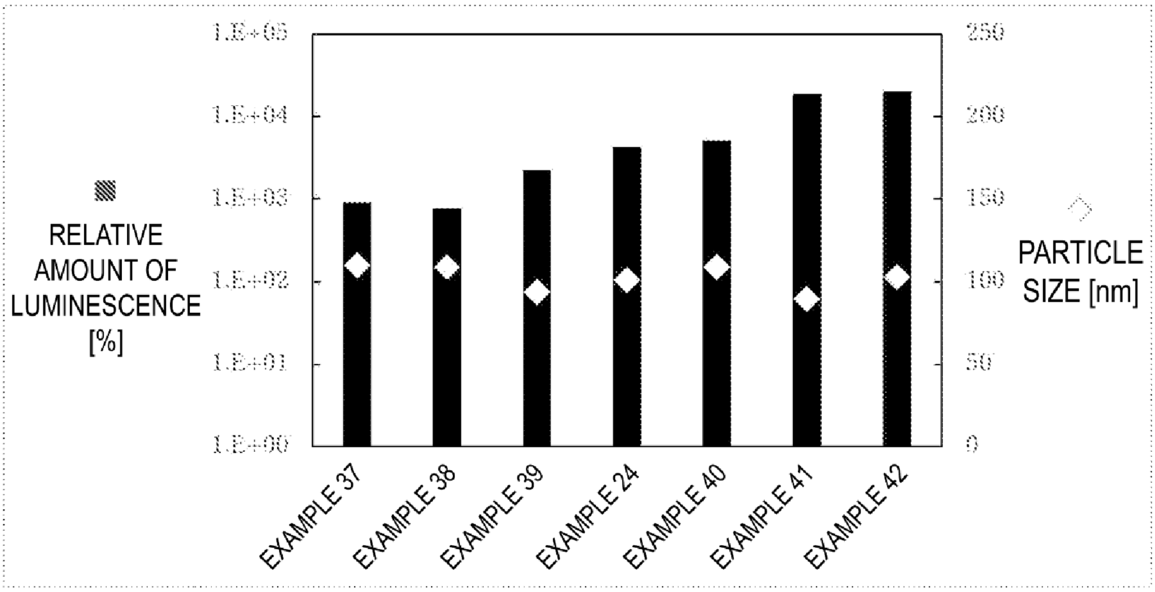
RELATIVE
AMOUNT OF
LUMINESCENCE
[%]
1.E+05
1.E+04
1.E+03
1.E+02
1.E+01
1.E+00
250
200
150
100
50
0
PARTICLE
SIZE [nm]
EXAMPLE 37
EXAMPLE 38
EXAMPLE 39
EXAMPLE 24
EXAMPLE 40
EXAMPLE 41
EXAMPLE 42

FIG. 8

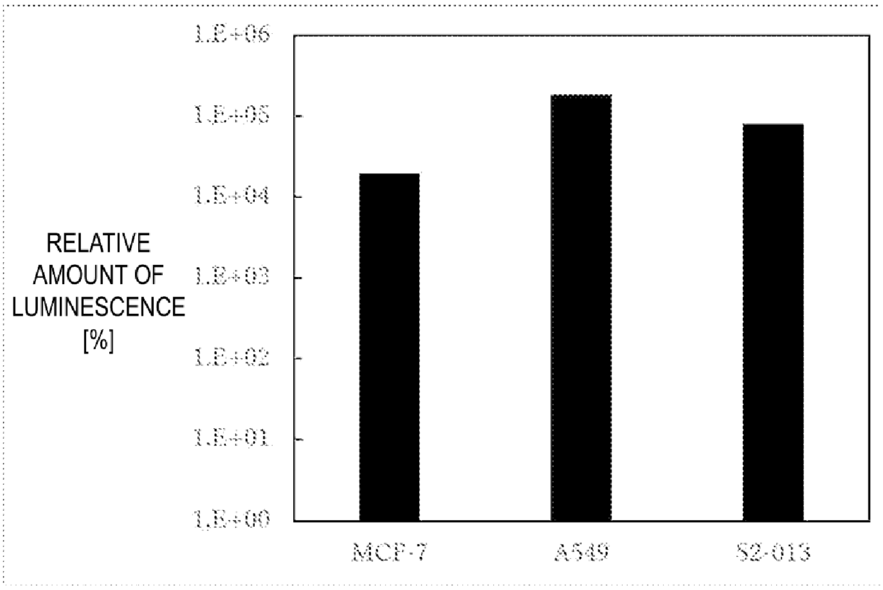
RELATIVE AMOUNT OF LUMINESCENCE [%]
1.E+06
1.E+05
1.E+04
1.E+03
1.E+02
1.E+01
1.E+00
MCF-7
A549
S2-013

FIG. 9

**INTERNATIONAL SEARCH REPORT**

International application No.
**PCT/JP2024/023226**

**A. CLASSIFICATION OF SUBJECT MATTER**

***A61K 9/16***(2006.01)i; ***A61K 31/713***(2006.01)i; ***A61K 31/7088***(2006.01)i; ***A61K 47/10***(2017.01)i; ***A61K 47/24***(2006.01)i; ***A61K 47/28***(2006.01)i; ***A61K 47/32***(2006.01)i
FI: A61K9/16 ZNA; A61K47/24; A61K47/28; A61K47/32; A61K47/10; A61K31/7088; A61K31/713

According to International Patent Classification (IPC) or to both national classification and IPC

**B. FIELDS SEARCHED**

Minimum documentation searched (classification system followed by classification symbols)

A61K9/16; A61K31/713; A61K31/7088; A61K47/10; A61K47/24; A61K47/28; A61K47/32

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/203998 A1 (NIPPON SHOKUBAI CO., LTD.) 08 October 2020 (2020-10-08) paragraphs [0074]-[0136] | 1-5, 8-11 |
| Y | paragraphs [0074]-[0136] | 6-7, 12 |
| Y | JP 2013-500960 A (RAMOT AT TEL-AVIV UNIVERSITY LTD.) 10 January 2013 (2013-01-10) paragraphs [0188], [0194] | 6-7, 12 |
| A | | 1-5, 8-11 |
| Y | JP 2007-106740 A (INDUSTRIAL TECHNOLOGY RESEARCH INSTITUTE) 26 April 2007 (2007-04-26) paragraphs [0056], [0066]-[0072] | 6-7, 12 |
| A | | 1-5, 8-11 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 September 2024** | **17 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/023226**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/023226**

| Patent document cited in search report | Publication date (day/month/year) | Patent family member(s) | Publication date (day/month/year) |
|---|---|---|---|
| WO 2020/203998 A1 | 08 October 2020 | EP 3950735 A1<br>paragraphs [0074]-[0129]<br>US 2022/185918 A1 | |
| JP 2013-500960 A | 10 January 2013 | US 2012/0129916 A1<br>paragraphs [0209], [0216]<br>EP 2459724 A2 | |
| JP 2007-106740 A | 26 April 2007 | US 2007/0087045 A1<br>paragraphs [0014], [0024]-[0034]<br>EP 1774962 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020532528 T
- JP 2010077091 A
- JP 2023121431 A